# EUROPEAN PATENT SPECIFICATION

(11) **EP 3 003 306 B1**
(45) Date of publication and mention of the grant of the patent: **26.08.2020**
(21) Application number: 14807186.3
(22) Date of filing: 04.06.2014
(51) Int. Cl.: A61K 31/436, A61K 39/395, A61K 48/00, A61P 19/02, A61P 37/00, A61K 38/13, A61K 39/00, A61K 39/39, A61K 9/127, A61K 9/51, C07K 14/575, C07K 16/18, C07K 14/47

(54) **REPEATED ADMINISTRATION OF NON-IMMUNOSUPRESSIVE ANTIGEN SPECIFIC IMMUNOTHERAPEUTICS**
WIEDERHOLTE VERABREICHUNG VON NICHT-IMMUNOSUPRESSIVEN ANTIGENSPEZIFISCHEN IMMUNTHERAPEUTIKA
ADMINISTRATION RÉPÉTÉE D'AGENTS IMMUNOTHÉRAPEUTIQUES SPÉCIFIQUES DE L'ANTIGÈNE NON IMMUNOSUPPRESSEURS

(30) Priority: 04.06.2013 US 201361831128 P
(43) Date of publication of application: 13.04.2016
(60) Divisional application: 20186198.6
(73) Proprietor: Selecta Biosciences, Inc., Watertown, MA 02472 (US)
(72) Inventor: MALDONADO, Roberto, A., Jamaica Plain, MA 02130 (US); KISHIMOTO, Takashi, Kei, Lexington, MA 02420 (US)
(74) Representative: EIP
(86) International application number: PCT/US2014/040938
(87) International publication number: WO 2014/197616

(56) References cited:
- WO-A1-2008/043157
- WO-A1-2010/056143
- WO-A2-2010/138192
- WO-A2-2012/054920
- WO-A2-2012/149255
- US-A1- 2004 005 315
- US-A1- 2011 020 388
- US-A1- 2012 076 831
- B. MOGHIMI ET AL: "Induction of tolerance to factor VIII by transient co-administration with rapamycin", JOURNAL OF THROMBOSIS AND HAEMOSTASIS, vol. 9, no. 8, 18 May 2011 (2011-05-18), pages 1524-1533, XP055091099, ISSN: 1538-7933, DOI: 10.1111/j.1538-7836.2011.04351.x
- ZHANG A.H.A. ET AL.: "Induction of tolerance to FVIII using nanoparticles in a murine model of hemophilia A", BLOOD, vol. 122, no. 21, 15 November 2013 (2013-11-15), page 2337,

## Description

### FIELD OF THE INVENTION

The technical field of the invention is the induction of antigen-specific tolerance.

### BACKGROUND

Certain diseases or conditions, such as autoimmune diseases, allergies, or genetic or acquired deficiencies requiring protein or enzyme replacement therapies, and diseases requiring biological therapies, often result in undesired immune responses. Such undesired immune responses may be reduced through the use of immunomodulator drugs. Conventional immunomodulator drugs, however, are broad-acting. Additionally, in order to maintain immunosuppression, immunomodulator drug therapy is generally a life-long proposition. Unfortunately, the use of broad-acting immunomodulators are associated with a risk of severe side effects, such as tumors, infections, nephrotoxicity and metabolic disorders. Accordingly, new immunomodulator therapies would be beneficial.

Moghimi (2011); J. Thromb. Haemost. 9(8):1524-1533 discloses the administration of Factor VIII and rapamycin. It is taught that tolerance for Factor VIII is induced, and that (at the doses used) immunosuppression is mild and transient.

Zhang (2013); Blood 122(21):2337 disclose nanoparticles with factor VIII and an immunomodulator, but the latter is not specified.

WO2008043157 discloses compositions comprising an antigen and an immunosuppressant in particulate form, and the use thereof in eliciting or stimulating tolerance.

WO2012054920 discloses a tolerogenic microparticle for treating diabetes.

### SUMMARY OF THE INVENTION

The invention is defined in the appended claims.

It is to be understood that the invention is not limited to what is presented in the examples by way of illustration.

### REFERENCE TECHNICAL DISCLOSURE

The reference technical disclosure set out below may in some respects go beyond the disclosure of the invention per se, and may also provide technical background for related technical developments, as illustrated inter alia in the reference examples. It will therefore be appreciated that this reference technical disclosure is not intended to define the invention as such, but rather to place it in a broader technical context.

This technical disclosure describes methods comprising determining a protocol for repeatedly administering an antigen-specific immunotherapeutic that does not result in immunosuppression in a subject; and administering repeatedly the antigen-specific immunotherapeutic to another subject using one or more elements of the protocol.

In another aspect of this technical disclosure, a method comprising determining a protocol for repeatedly administering an antigen-specific immunotherapeutic that does not result in immunosuppression in a subject, wherein the determining comprises administering repeatedly the antigen-specific immunotherapeutic to a subject is described.

In another aspect of this technical disclosure, described is a method comprising repeatedly administering to a subject an antigen-specific immunotherapeutic that comprises an antigen or an immunomodulator, wherein the antigen or immunomodulator is repeatedly administered according to one or more elements of a protocol that does not induce immunosuppression upon repeated administration of the antigen or an immunomodulator.

In one aspect of this technical disclosure, the method further comprises obtaining or providing an antigen-specific immunotherapeutic. In another aspect of this technical disclosure, the determining further comprises demonstrating that one or more elements of the protocol results in antigen-specific tolerance in the subject.

In another aspect of this technical disclosure, a composition comprising an antigen-specific immunotherapeutic that comprises an exogenous antigen or an exogenous immunomodulator in an amount previously demonstrated in a protocol not to induce immunosuppression upon repeated administration is described. In one aspect of this technical disclosure, the composition is a kit.

In one aspect of this technical disclosure, the antigen-specific immunotherapeutic is any one of the antigen-specific immunotherapeutics as described herein.

In another aspect of this technical disclosure, the protocol is one that has been previously shown not to induce immunosuppression.

In another aspect of this technical disclosure, the antigen or immunomodulator is present in an amount further shown to result in antigen-specific tolerance.

In another aspect of this technical disclosure, the antigen-specific immunotherapeutic comprises an exogenous immunomodulator. In another aspect of this technical disclosure, the exogenous immunomodulator comprises alan statin; mTOR inhibitor; TGF-β signaling agent; TGF-β receptor agonist; histone deacetylase inhibitor; corticosteroid; inhibitor of mitochondrial function; P38 inhibitor; NF-κβ inhibitor; lectin receptor ligand; adenosine receptor agonist; prostaglandin E2 agonist; phosphodiesterase inhibitor; proteasome inhibitor; kinase inhibitor; G-protein coupled receptor agonist; G-protein coupled receptor antagonist; glucocorticoid; retinoid; cytokine inhibitor; cytokine receptor inhibitor; cytokine receptor activator; peroxisome proliferator-activated receptor antagonist; peroxisome proliferator-activated receptor agonist; histone deacetylase inhibitor; calcineurin inhibitor; phosphatase inhibitor; oxidized ATP; IDO; vitamin D3; cyclosporine A; aryl hydrocarbon receptor inhibitor; resveratrol; azathiopurine; 6-mercaptopurine; aspirin; niflumic acid; estriol; tripolide; interleukin; cyclosporine A, or siRNA targeting cytokines or cytokine receptors. In another case, the exogenous immunomodulator comprises rapamycin, mycophenolic acid or a CD22 ligand.

In another aspect of this technical disclosure, the antigen-specific immunotherapeutic comprises an exogenous antigen.

In another aspect of this technical disclosure, when the antigen-specific immunotherapeutic also comprises an exogenous immunomodulator, the exogenous antigen and exogenous immunomodulator are not coupled to each other. In another aspect of this technical disclosure, the repeated administration comprises concomitant repeated administration of the exogenous antigen and exogenous immunomodulator.

In another aspect of this technical disclosure, the exogenous antigen comprises a therapeutic protein, modified antigen or expressed antigen. In another aspect of this technical disclosure, the expressed antigen is expressed from modified messenger RNA.

In another aspect of this technical disclosure, the antigen-specific immunotherapeutic results in antigen-specific tolerance to an endogenous antigen.

In another aspect of this technical disclosure, the endogenous antigen comprise an autoantigen.

In another aspect of this technical disclosure, the autoantigen comprises those found in Anklosing spondylitis; bulous pemiphigous; rheumatoid arthritis; multiple sclerosis; diabetes; excema; inflammatory bowel disease; lupus or systemic lupus erythematosus; multiple sclerosis; primary biliary cirrhosis; psoriasis; sarcoidosis; systemic sclerosis; scleroderma; thyroiditis; autoimmune thyroid disease; Hashimoto's thyroiditis; thyrotoxicosis; alopecia areata; Grave's disease; Guillain-Barre syndrome; celiac disease; Sjögren's syndrome; rheumatic fever; gastritis autoimmune atrophic gastritis; autoimmune hepatitis; insulitis; oophoritis; orchitis; uveitis; phacogenic uveitis; myasthenia gravis; primary myxoedema; pernicious anemia; primary sclerosing cholangitis; autoimmune haemolytic anemia; Addison's disease; scleroderma; Goodpasture's syndrome; nephritis; psoriasis; pemphigus vulgaris; pemphigoid; sympathetic opthalmia; idiopathic thrombocylopenic purpura; idiopathic feucopenia; Wegener's granulomatosis or poly/dermatomyositis.

In another aspect of this technical disclosure, the antigen-specific immunotherapeutic comprises an exogenous antigen and results in antigen-specific tolerance when administered in the presence of an endogenous immunomodulator.

In another aspect of this technical disclosure, the endogenous immunomodulator comprises a substance and/or combination of substances involved in apoptosis or related signalling, a substance and/or combination of substances involved in T or B cell biology, or a substance and/or combination of substances involved in dendritic cell biology.

In another aspect of this technical disclosure, the repeated administration occurs 1 week to 10 years after an initial dose or a previous repeated administration of the antigen-specific immunotherapeutic. In another embodiment of any one of the methods or compositions described herein, the repeated administration occurs 1 week after an initial dose or a previous repeated administration of the antigen-specific immunotherapeutic. In another aspect of this technical disclosure, the repeated administration occurs 2 weeks after an initial dose or a previous repeated administration of the antigen-specific immunotherapeutic. In another aspect of this technical disclosure, the repeated administration occurs 1 to 12 months after an initial dose or a previous repeated administration of the antigen-specific immunotherapeutic.

In another aspect of this technical disclosure, the antigen-specific immunotherapeutic comprises an exogenous antigen and exogenous immunomodulator, the exogenous antigen is repeatedly administered by a route different from the exogenous immunomodulator. In another aspect of this technical disclosure, repeated administration comprises concomitant repeated administration.

In another aspect of this technical disclosure, the antigen-specific immunotherapeutic comprises more than one exogenous antigen.

In another aspect of this technical disclosure, when the antigen-specific immunotherapeutic comprises an exogenous antigen and exogenous immunomodulator, the exogenous antigens are repeatedly administered by a route different from the exogenous immunomodulator. In another aspect of this technical disclosure, the repeated administration comprises concomitant repeated administration.

In another aspect of this technical disclosure, the exogenous antigen and exogenous immunomodulator are coupled to each other. In another aspect of this technical disclosure, the exogenous immunomodulator comprises ERY1 peptide.

In another aspect of this technical disclosure, the antigen-specific immunotherapeutic is repeatedly administered to another subject using all or substantially all of the elements of the protocol.

In another aspect of this technical disclosure, the antigen-specific immunotherapeutic comprises polymeric synthetic nanocarriers coupled to an exogenous immunomodulator.

In another aspect of this technical disclosure, a load of immunomodulator attached to the polymeric synthetic nanocarriers, on average across the polymeric synthetic nanocarriers, is between 0.1% and 50% (weight/weight). In another aspect of this technical disclosure, the load is between 0.1% and 20% (weight/weight).

In another aspect of this technical disclosure, the load of immunomodulator of the exogenous immunodulator on average is at least 95%, 97%, 98% or 99% (weight/weight).

In another aspect of this technical disclosure, when the antigen-specific immunotherapeutic comprises an exogenous immunomodulator and exogenous antigen, the polymeric synthetic nanocarriers are further coupled to the exogenous antigen. In another aspect of this technical disclosure, when the antigen-specific immunotherapeutic comprises an exogenous immunomodulator and exogenous antigen, the polymeric synthetic nanocarrier is concomitantly administered with an exogenous antigen.

In another aspect of this technical disclosure, a method of manufacturing any one of the antigen-specific immunotherapeutics described herein is disclosed. In one aspect of this technical disclosure, the method comprises producing or obtaining an exogenous antigen or an exogenous immunomodulator in an amount that does not induce immunosuppression upon repeated administration. In another aspect of this technical disclosure, the amount is in an amount previously demonstrated in a protocol not to induce immunosuppression upon repeated administration. In another aspect of this technical disclosure, the method further comprises determining the amount or the protocol.

In another aspect of this technical disclosure, an antigen-specific immunotherapeutic comprising an exogenous immunomodulator or an exogenous antigen for the manufacture of a medicament for achieving antigen-specific tolerance but not induction of immunosuppression in a subject is described.

In another aspect of this technical disclosure, an antigen-specific immunotherapeutic comprising an exogenous immunomodulator or an exogenous antigen, for achieving antigen-specific tolerance but not induction of immunosuppression in a subject is described. In one aspect of this technical disclosure, the antigen-specific immunotherapeutic is for use in any one of the methods described herein.

In another aspect of this technical disclosure, the antigen-specific immunotherapeutic is any one of the antigen-specific immunotherapeutics described herein.

### BRIEF DESCRIPTION OF FIGURES

**Fig. 1** shows results from repeated administration of an antigen-specifc immunotherapeutic comprising antigen and rapamycin.
**Fig. 2** shows results from repeated administration of an antigen-specific immunotherapeutic comprising methotrexate, an exogenous immunomodulator.
**Fig. 3** shows results from repeated administration of an antigen-specific immunotherapeutic comprising methotrexate, an exogenous immunomodulator.
**Fig. 4** demonstrates the deletion or anergy of CD8⁺ T cells with an exogenous antigen (OVA) attached to exogenous immunomodulator (ERY1 peptide).

### DETAILED DESCRIPTION

The detailed reference technical disclosure set out below may in some respects go beyond the disclosure of the invention per se, and may also provide technical background for related technical developments, as illustrated inter alia in the reference examples. It will therefore be appreciated that this reference technical disclosure is not intended to define the invention as such, but rather to place it in a broader technical context.

As used in this specification and the appended claims, the singular forms "a," "an" and "the" include plural referents unless the content clearly dictates otherwise. For example, reference to "a polymer" includes a mixture of two or more such molecules or a mixture of differing molecular weights of a single polymer species, reference to "a synthetic nanocarrier" includes a mixture of two or more such synthetic nanocarriers or a plurality of such synthetic nanocarriers, reference to "a DNA molecule" includes a mixture of two or more such DNA molecules or a plurality of such DNA molecules, reference to "an immunomodulator" includes a mixture of two or more such materials or a plurality of immunomodulator molecules.

As used herein, the term "comprise" or variations thereof such as "comprises" or "comprising" are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein, the term "comprising" is inclusive and does not exclude additional, unrecited integers or method/process steps.

In any of the compositions and methods described herein, "comprising" may be replaced with "consisting essentially of' or "consisting of'. The phrase "consisting essentially of' is used herein to require the specified integer(s) or steps as well as those which do not materially affect the character or function of the claimed invention. As used herein, the term "consisting" is used to indicate the presence of the recited integer (e.g. a feature, element, characteristic, property, method/process step or limitation) or group of integers (e.g. features, element, characteristics, properties, method/process steps or limitations) alone.

### A. INTRODUCTION

As previously mentioned, current conventional immunomodulating compositions are broad acting and generally result in an overall systemic downregulation of the immune system. The compositions and methods described herein allow for more targeted immune effects, particularly when the recited antigen-specific immunotherapeutics are used in repeated administration. Broad immunosuppression during repeated administration is of particular concern, because it generally would result in long-term immunosuppression that could lead to significant adverse events for the subjects receiving the repeatedly administered conventional immunomodulating compositions. Instead, the inventors have discovered that it is possible to provide antigen-specific immunomodulatory compositions and methods that do not result in long-term or broad immunosuppression during repeated administration.

The inventors have unexpectedly and surprisingly discovered that the problems and limitations noted above can be overcome by practicing the invention disclosed herein. In particular, the inventors have unexpectedly discovered that it is possible to provide methods comprising determining a protocol for repeatedly administering an antigen-specific immunotherapeutic that does not result in immunosuppression in a subject; and administering repeatedly the antigen-specific immunotherapeutic to another subject using one or more elements of the protocol. Additionally, the inventors have unexpectedly discovered that it is possible to provide methods comprising: repeatedly administering to a subject an antigen-specific immunotherapeutic that comprises an antigen or an immunomodulator, wherein the antigen or immunomodulator is repeatedly administered according to one or more elements of a protocol that does not induce immunosuppression upon repeated administration of the antigen or immunomodulator. In some embodiments, the protocol is one that has been previously shown not to induce immunosuppression in a subject. Further, the inventors have unexpectedly discovered that it is possible to provide compositions comprising: an antigen-specific immunotherapeutic that comprises an exogenous antigen or an exogenous immunomodulator in an amount that does not induce immunosuppression when repeatedly administered. In some embodiments, the amount is one that has been previously demonstrated in a protocol not to induce immunosuppression upon repeated administration in a subject.

### B. DEFINITIONS

"Administering" or "administration" or "administer" means providing a material to a subject in a manner that is pharmacologically useful. The term is intended to include causing to be administered in some cases. "Causing to be administered" means causing, urging, encouraging, aiding, inducing or directing, directly or indirectly, another party to administer the material.

"An amount previously demonstrated in a protocol not to induce immunosuppression upon repeated administration" in the context of a composition, dosage form, or method for administration to a subject refers to an amount of the antigen or immunomodulator that does not induce immunosuppression upon repeated administration when administered according to a protocol previously demonstrated shown not to induce immunosuppression

Amounts effective will depend, of course, on the particular subject being treated; the severity of a condition, disease or disorder; the individual patient parameters including age, physical condition, size and weight; the duration of the treatment; the nature of concurrent therapy (if any); the specific route of administration and like factors within the knowledge and expertise of the health practitioner. These factors are well known to those of ordinary skill in the art and can be addressed with no more than routine experimentation. It is generally preferred that a maximum dose be used, that is, the highest safe dose according to sound medical judgment. It will be understood by those of ordinary skill in the art, however, that a patient may insist upon a lower dose or tolerable dose for medical reasons, psychological reasons or for virtually any other reason.

In certain cases, doses or amounts of the immunomodulators and/or antigens in the compositions can range from about 10 µg/kg to about 100,000 µg/kg. In some cases, the doses can range from about 0.1 mg/kg to about 100 mg/kg. In still other cases, the doses can range from about 0.1 mg/kg to about 25 mg/kg, about 25 mg/kg to about 50 mg/kg, about 50 mg/kg to about 75 mg/kg or about 75 mg/kg to about 100 mg/kg. Alternatively, the dose or amount can be administered based on the number of synthetic nanocarriers that provide the desired amount of immunomodulators and/or antigens. For example, useful doses or amounts include greater than 10⁶, 10⁷, 10⁸, 10⁹ or 10¹⁰ synthetic nanocarriers (per dose). Other examples of useful doses or amounts include from about 1x10⁶ to about 1x10¹⁰, about 1x10⁷ to about 1x10⁹ or about 1x10⁸ to about 1x10⁹ synthetic nanocarriers (per dose).

"Antigen" means a B cell antigen or T cell antigen. "Type(s) of antigens" means molecules that share the same, or substantially the same, antigenic characteristics. In some cases, antigens may be proteins, polypeptides, peptides, lipoproteins, glycolipids, polynucleotides, polysaccharides or are contained or expressed in cells. In some cases, such as when the antigens are not well defined or characterized, the antigens may be contained within a cell or tissue preparation, cell debris, cell exosomes, conditioned media, etc. In some cases, the antigen can be endogenous or exogenous. Endogenous antigen comprises antigen that is generated by a subject's own body, and can result in immune responses that can lead to antigen-specific tolerance with an antigen-specific immunotherapeutic, such as one that comprises exogenous immunomodulator, upon repeated administration as described herein. In some cases, the endogenous antigen results in antigen-specific tolerance with the repeated administration of an exogenous immunomodulator as described herein. Examples of endogenous antigen comprise autoimmune antigens, some of which are disclosed elsewhere herein. Exogenous antigen comprises antigen that is administered as part of the antigen-specific immunotherapeutic or as part of some other therapeutic intervention, but is not generated by a subject's own body. Examples of exogenous antigens comprise environmental allergens, therapeutic proteins or polypeptides, etc. some of which are disclosed elsewhere herein.

"Antigen-specific" refers to any immune response that results from the presence of the antigen, or portion thereof, or that generates molecules that specifically recognize or bind the antigen. For example, where the immune response is antigen-specific antibody production, antibodies are produced that specifically bind the antigen. As another example, where the immune response is antigen-specific B cell or CD4+ T cell or CD8+ T cell activation, proliferation and/or activity, the activation, proliferation and/or activity results from recognition of the antigen, or portion thereof, alone or in complex with MHC molecules, B cells, etc.

"Antigen-specific immunotherapeutic" means a therapeutic agent that is capable of having a tolerogenic effect on a subject's immune response to an antigen of interest. An antigen-specific immunotherapeutic can comprise an antigen or an immunomodulator. In certain cases, antigen-specific immunotherapeutics can comprise both an antigen and an immunomodulator, wherein the antigen is coupled or uncoupled to the immunomodulator. In certain cases, antigen-specific immunotherapeutics can comprise an antigen and an immunomodulator that are not coupled to each other and the antigen and immunomodulator are repeatedly administered concomitantly. In such cases, the antigen and immunomodulator may be administered in the same composition or as separate compositions, and it is the totality of the compositions comprising the antigen or immunomdoulator that constitutes the antigen-specific immunotherapeutic. In cases, antigen-specific immunotherapeutics that comprise antigens (i.e. exogenous antigens) and/or immunomodulators (i.e. exogenous immunomodulators) may interact with endogenous immunomodulators and/or endogenous antigens, respectively, to preferably result in or lead to immune responses that can result in antigen-specific tolerance.

"Antigen-specific immunotherapeutic efficacy" means that, for an antigen of interest (Agi) the Agi IgG titer (reported as EC50) changes from level of positive control to a titer (reported as EC50) at least 50% lower, with same Agi dosing. See generally J. R. Crowther, "ELISA: Theory and Practice" (1995 Humana Press).

"Average", as used herein, refers to the arithmetic mean unless otherwise noted.

"B cell antigen" means any antigen that is recognized by and triggers an immune response in a B cell (e.g., an antigen that is specifically recognized by a B cell or a receptor thereon). In some cases, an antigen that is a T cell antigen is also a B cell antigen. In other cases, the T cell antigen is not also a B cell antigen. B cell antigens include, but are not limited to proteins, peptides, etc. In some cases, the B cell antigen comprises a non-protein antigen (i.e., not a protein or peptide antigen).

"Causing" means to make an action happened either directly or indirectly (for example through a third party). In some cases, the invention finds application in methods which comprise causing the antigen-specific immunotherapeutic to be repeatedly administered to another subject using one or more elements of the protocol.

"Combination", as applied to two or more materials and/or agents (also referred to herein as the *components*), is intended to define material in which the two or more materials /agents are associated. Components may separately identified, e.g. first component, second component, third component, etc. The terms "combined" and "combining" in this context are to be interpreted accordingly.

The association of the two or more materials /agents in a combination may be physical or non-physical. Examples of physically associated combined materials/agents include:
- compositions (e.g. unitary formulations) comprising the two or more materials/agents in admixture (for example within the same unit dose);
- compositions comprising material in which the two or more materials/agents are chemically/physicochemically linked (for example by crosslinking, molecular agglomeration or binding to a common vehicle moiety);
- compositions comprising material in which the two or more materials/agents are chemically/physicochemically co-packaged (for example, disposed on or within lipid vesicles, particles (e.g. micro- or nanoparticles) or emulsion droplets);
- pharmaceutical kits, pharmaceutical packs or patient packs in which the two or more materials/agents are co-packaged or co-presented (e.g. as part of an array of unit doses);

Examples of non-physically associated combined materials/agents include:
- material (e.g. a non-unitary formulation) comprising at least one of the two or more materials/agents together with instructions for the extemporaneous association of the at least one compound/agent to form a physical association of the two or more materials/agents;
- material (e.g. a non-unitary formulation) comprising at least one of the two or more materials/agents together with instructions for combination therapy with the two or more materials/agents;
- material comprising at least one of the two or more materials/agents together with instructions for administration to a patient population in which the other(s) of the two or more materials/agents have been (or are being) administered;
- material comprising at least one of the two or more materials/agents in an amount or in a form which is specifically adapted for use in combination with the other(s) of the two or more materials/agents.

As used herein, the term "combination therapy" is intended to define therapies which comprise the use of a combination of two or more materials/agents (as defined herein). Thus, references to "combination therapy", "combinations" and the use of materials/agents "in combination" in this application may refer to materials/agents that are administered as part of the same overall treatment regimen. As such, the posology of each of the two or more materials/agents may differ: each may be administered at the same time or at different times. It will therefore be appreciated that the materials/agents of the combination may be administered sequentially (e.g. before or after) or simultaneously, either in the same pharmaceutical formulation (i.e. together), or in different pharmaceutical formulations (i.e. separately). Simultaneously in the same formulation is as a unitary formulation whereas simultaneously in different pharmaceutical formulations is non-unitary. The posologies of each of the two or more materials/agents in a combination therapy may also differ with respect to the route of administration.

"Concomitantly" means administering two or more materials/agents to a subject in a manner that is correlated in time, preferably sufficiently correlated in time so as to provide a modulation in an immune response, and even more preferably the two or more materials/agents are administered in combination. In some cases, concomitant administration may encompass administration of two or more materials/agents within a specified period of time, preferably within 1 month, more preferably within 1 week, still more preferably within 1 day, and even more preferably within 1 hour. In some cases, the materials/agents may be repeatedly administered concomitantly; that is concomitant administration on more than one occasion.

"Couple" or "Coupled" or "Couples" (and the like) means to chemically associate one entity (for example a moiety) with another. In some cases, the coupling is covalent, meaning that the coupling occurs in the context of the presence of a covalent bond between the two entities. In non-covalent cases, the non-covalent coupling is mediated by non-covalent interactions including but not limited to charge interactions, affinity interactions, metal coordination, physical adsorption, host-guest interactions, hydrophobic interactions, TT stacking interactions, hydrogen bonding interactions, van der Waals interactions, magnetic interactions, electrostatic interactions, dipole-dipole interactions, and/or combinations thereof. In some cases, encapsulation is a form of coupling.

"Determining" or "determine" or "demonstrating" or "demonstrate" means to ascertain a factual relationship. These terms mean establishing a connection between one or more inputs, for example the elements of a protocol or the entire protocol, and one or more outputs, for example the presence or absence of immunosuppression or the achievement of antigen-specific tolerance. In some cases, determining that one or more elements of a protocol for repeatedly administering an antigen-specific immunotherapeutic do not result in immunosuppression in a subject is envisaged.

Determining, etc. may be accomplished in a number of ways, including but not limited to performing experiments, or making projections. For instance, one or more elements of a protocol, such as a dose of an immunomodulator, may be determined by starting with one or more elements of a test protocol, such as a test dose, and using known scaling techniques (such as allometric or isometric scaling) to determine the protocol, such as the dose, for administration. In another case, one or more elements of a protocol, such as a dose, may be determined by testing variations in the one or more elements, such as various doses in a subject, e.g. through direct experimentation based on experience and guiding data. In some cases, "determining" or "determine" or "demonstrating" or "demonstrate" comprises "causing to be determined" "or causing to be demonstrated". "Causing to be determined" "or causing to be demonstrated" means causing, urging, encouraging, aiding, inducing or directing or acting in coordination with an entity for the entity to ascertain a factual relationship; including directly or indirectly, or expressly or impliedly.

"Dosage form" means a pharmacologically and/or immunologically active material in a medium, carrier, vehicle, or device suitable for administration to a subject. Any one of the compositions or doses described herein may be in a dosage form.

"Encapsulate" means to enclose at least a portion of a substance within a synthetic nanocarrier. In some cases, a substance is enclosed completely within a synthetic nanocarrier. In other cases, most or all of a substance that is encapsulated is not exposed to the local environment external to the synthetic nanocarrier. In other cases, no more than 50%, 40%, 30%, 20%, 10% or 5% (weight/weight) is exposed to the local environment. Encapsulation is distinct from absorption, which places most or all of a substance on a surface of a synthetic nanocarrier, and leaves the substance exposed to the local environment external to the synthetic nanocarrier.

"Immunomodulator" means a compound or combination of compounds that causes an APC (Antigen Presenting Cell) to have a tolerogenic effect. A tolerogenic effect generally refers to the production or expression of cytokines or other factors by the APC or changes in the genetic expression profile of the APCs (e.g, changes in co-stimulatory molecule expression) that reduces, inhibits or prevents an undesired antigen-specific immune response or that promotes a desired antigen-specific tolerogenic immune response. In some cases, the immunomodulator can be endogenous or exogenous. Endogenous immunomodulators comprise immunomodulators that are generated by a subject's own body, and can result in immune responses that can lead to antigen-specific tolerance with an antigen-specific immunotherapeutic, such as one comprising exogenous antigen, upon repeated administration as described herein. In some cases, the endogenous immunomodulator can result in antigen-specific tolerance when an exogenous antigen is administered as described herein. Examples of endogenous immunomodulators comprise apoptotic cells and other apoptotic ligands or markers, tolerogenic cytokines such as IL-10, and cell surface markers implicated in tolerogenic responses such as CD22. Exogenous immunomodulators comprise immunomodulators that are administered as part of the antigen-specific immunotherapeutic or as part of some other therapeutic intervention, but are not generated by a subject's own body. Examples of exogenous immunomodulators comprise rapamcycin and other immunomodulators disclosed herein.

In one case, the immunomodulator is one that causes an APC to promote a regulatory phenotype in one or more immune effector cells. For example, the regulatory phenotype may be characterized by the inhibition of the production, induction, stimulation or recruitment of antigen-specific CD4+ T cells or B cells, the inhibition of the production of antigen-specific antibodies, the production, induction, stimulation or recruitment of Treg cells (e.g., CD4+CD25highFoxP3+ Treg cells), etc. This may be the result of the conversion of CD4+ T cells or B cells to a regulatory phenotype. This may also be the result of induction of FoxP3 in other immune cells, such as CD8+ T cells, macrophages and iNKT cells. In one case, the immunomodulator is one that affects the response of the APC after it processes an antigen. In another case, the immunomodulator is not one that interferes with the processing of the antigen. In a further case, the immunomodulator is not an apoptotic-signaling molecule. In another case, the immunomodulator is not a phospholipid.

Immunomodulators include, but are not limited to, statins; mTOR inhibitors, such as rapamycin or a rapamycin analog; TGF-β signaling agents; TGF-β receptor agonists; histone deacetylase inhibitors, such as Trichostatin A; corticosteroids; inhibitors of mitochondrial function, such as rotenone; P38 inhibitors; NF-κβ inhibitors, such as 6Bio, Dexamethasone, TCPA-1, IKK VII; adenosine receptor agonists; prostaglandin E2 agonists (PGE2), such as Misoprostol; phosphodiesterase inhibitors, such as phosphodiesterase 4 inhibitor (PDE4), such as Rolipram; proteasome inhibitors; kinase inhibitors; G-protein coupled receptor agonists; G-protein coupled receptor antagonists; glucocorticoids; retinoids; cytokine inhibitors; cytokine receptor inhibitors; cytokine receptor activators; peroxisome proliferator-activated receptor antagonists; peroxisome proliferator-activated receptor agonists; histone deacetylase inhibitors; calcineurin inhibitors; phosphatase inhibitors; PI3KB inhibitors, such as TGX-221; autophagy inhibitors, such as 3-Methyladenine; aryl hydrocarbon receptor inhibitors; proteasome inhibitor I (PSI); and oxidized ATPs, such as P2X receptor blockers. Immunomodulators also include IDO, vitamin D3, cyclosporins, such as cyclosporine A, aryl hydrocarbon receptor inhibitors, resveratrol, azathiopurine (Aza), 6-mercaptopurine (6-MP), 6-thioguanine (6-TG), FK506, sanglifehrin A, salmeterol, mycophenolate mofetil (MMF), aspirin and other COX inhibitors, niflumic acid, estriol and triptolide. In some cases, the immunomodulator may comprise any one of the agents described herein.

The immunomodulator can be a compound that directly provides the tolerogenic effect on APCs or it can be a compound that provides the tolerogenic effect indirectly (i.e., after being processed in some way after administration). Immunomodulators, therefore, include prodrug forms of any of the compounds described herein.

Immunomodulators also include nucleic acids that encode the peptides, polypeptides or proteins described herein that result in a tolerogenic immune response. In some cases, therefore, the immunomodulator is a nucleic acid that encodes a peptide, polypeptide or protein that results in a tolerogenic immune response, and it is the nucleic acid that is coupled to the synthetic nanocarrier.

The nucleic acid may be DNA or RNA, such as mRNA. In some cases, the compositions comprise a complement, such as a full-length complement, or a degenerate (due to degeneracy of the genetic code) of any of the nucleic acids described herein. In come cases, the nucleic acid is an expression vector that can be transcribed when transfected into a cell line. In some cases, the expression vector may comprise a plasmid amongst others. Nucleic acids can be isolated or synthesized using standard molecular biology approaches, for example by using a polymerase chain reaction to produce a nucleic acid fragment, which is then purified and cloned into an expression vector. Additional techniques useful in the practice of this invention may be found in Current Protocols in Molecular Biology 2007 by John Wiley and Sons, Inc.; Molecular Cloning: A Laboratory Manual (Third Edition) Joseph Sambrook, Peter MacCallum Cancer Institute, Melbourne, Australia; David Russell, University of Texas Southwestern Medical Center, Dallas, Cold Spring Harbor.

In some cases, the immunomodulators described herein are coupled to synthetic nanocarriers. In preferable cases, the immunomodulator is an element that is in addition to the material that makes up the structure of the synthetic nanocarrier. For example, in one case, where the synthetic nanocarrier is made up of one or more polymers, the immunomodulator is a compound that is in addition and coupled to the one or more polymers. As another example, in one case, where the synthetic nanocarrier is made up of one or more lipids, the immunomodulator is again in addition and coupled to the one or more lipids. In some cases, such as where the material of the synthetic nanocarrier also results in a tolerogenic effect, the immunomodulator is an element present in addition to the material of the synthetic nanocarrier that results in a tolerogenic effect.

Other exemplary immunomodulators include, but are not limited, small molecule drugs, natural products, antibodies (e.g., antibodies against CD20, CD3, CD4), biologics-based drugs, carbohydrate-based drugs, nanoparticles, liposomes, RNAi, antisense nucleic acids, aptamers, methotrexate, NSAIDs; fingolimod; natalizumab; alemtuzumab; anti-CD3; tacrolimus (FK506), etc. Further immunomodulators, are known to those of skill in the art.

In some of the methods or compositions described herein, the immunomodulator is in a form, such as a nanocrystalline form, whereby the form of the immunomodulator itself is a particle or particle-like. In some cases, such forms mimic a virus or other foreign pathogen. Many drugs have been nanonized and appropriate methods for producing such drug forms would be known to one of ordinary skill in the art. Drug nanocrystals, such as nanocrystalline rapamycin are known to those of ordinary skill in the art (Katteboinaa, et al. 2009, International Journal of PharmTech Resesarch; Vol. 1, No. 3; pp682-694. As used herein a "drug nanocrystal" refers to a form of a drug (e.g., an immunomodulator) that does not include a carrier or matrix material. In some cases, drug nanocrystals comprise 90%, 95%, 98%, or 99% or more drug. Methods for producing drug nanocrystals include, without limitation, milling, high pressure homogenization, precipitation, spray drying, rapid expansion of supercritical solution (RESS), Nanoedge® technology (Baxter Healthcare), and Nanocrystal Technology™ (Elan Corporation). In some cases, a surfactant or a stabilizer may be used for steric or electrostatic stability of the drug nanocrystal. In some cases the nanocrystal or nanocrytalline form of an immunomodulator may be used to increase the solubility, stability, and/or bioavailability of the immunomodulator, particularly immunomodulators that are insoluble or labile.

"Immunosuppression" means (1) non-durable statistically-significant downregulation of an immune response as a result of repeated administration of an antigen-specific immunotherapeutic, or (2) the response of a non-human test subject to a KLH challenge T-cell dependent antibody response ELISA assay, assuming that KLH is not the antigen of interest, following at least one repeated administration of an antigen-specific immunotherapeutic, wherein the response is characterized as the KLH IgG titer (reported as EC50) changing from level of positive control to a titer (reported as EC50) equivalent to, or less than, 3 standard deviations above the mean negative control ("background"), with same KLH dosing. See generally J. R. Crowther, "ELISA: Theory and Practice" (1995 Humana Press). In a preferred case, non-durable statistically-significant downregulation means that the downregulation (treatment arm measured against non-treatment arm) does not evidence a statistically-significant difference for longer than a week following the last repeated administration of the antigen-specific immunotherapeutic. Various compositions, methods, protocols, and dosages forms do not result in, or do not induce, immunosuppression.

KLH challenge ELISA assays are described generally in the literature, for example in J.T. Brisbin et al., Influence of In-Feed Virginiamycin on the Systemic and Mucosal Antibody Response of Chickens, Poultry Science 87:1995-1999 (2008); or may be obtained commercially, for example from Stellar Biotechnologies (332 East Scott Street, Port Hueneme, California 93041 USA) as Item ELI-01G Mouse Anti-KLH IgG ELISA Kit, or ELI-03G NHP Anti-KLH IgG ELISA Kit.

An ELISA method for measuring an anti-KLH antibody titer, for example, a typical sandwich ELISA, may consist of the following steps (i) preparing an ELISA-plate coating material such that the antibody target of interest is coupled to a substrate polymer or other suitable material (ii) preparing the coating material in an aqueous solution (such as PBS) and delivering the coating material solution to the wells of a multiwell plate for overnight deposition of the coating onto the multiwell plate (iii) thoroughly washing the multiwell plate with wash buffer (such as 0.05% Tween-20 in PBS) to remove excess coating material (iv) blocking the plate for nonspecific binding by applying a diluent solution (such as 10% fetal bovine serum in PBS), (v) washing the blocking/diluent solution from the plate with wash buffer (vi) diluting the serum sample(s) containing antibodies and appropriate standards (positive controls) with diluent as required to obtain a concentration that suitably saturates the ELISA response (vii) serially diluting the plasma samples on the multiwell plate such to cover a range of concentrations suitable for generating an ELISA response curve (viii) incubating the plate to provide for antibody-target binding (ix) washing the plate with wash buffer to remove antibodies not bound to antigen (x) adding an appropriate concentration of a secondary detection antibody in same diluent such as a biotin-coupled detection antibody capable of binding the primary antibody (xi) incubating the plate with the applied detection antibody, followed by washing with wash buffer (xii) adding an enzyme such as streptavidin-HRP (horse radish peroxidase) that will bind to biotin found on biotinylated antibodies and incubating (xiii) washing the multiwell plate (xiv) adding substrate(s) (such as TMB solution) to the plate (xv) applying a stop solution (such as 2N sulfuric acid) when color development is complete (xvi) reading optical density of the plate wells at a specific wavelength for the substrate (450 nm with subtraction of readings at 570 nm) (xvi) applying a suitable multiparameter curve fit to the data and defining half-maximal effective concentration (EC50) as the concentration on the curve at which half the maximum OD value for the plate standards is achieved.

"Load" is the amount of the immunomodulator of an exogenous immunomodulator composition (weight/weight). For example, when attached to a synthetic nanocarrier, the load is based on the total dry recipe weight of materials in an entire synthetic nanocarrier (weight/weight). Generally, such a load is calculated as an average across a population of synthetic nanocarriers. In one case, the load on average across the synthetic nanocarriers is between 0.1% and 99%. In another case, the load is between 0.1% and 50%. In another case, the load of the immunomodulator is between 0.1% and 20%. In another case, the load of the immunomodulator is no more than 25% on average across a population of synthetic nanocarriers. In some cases, the load is calculated as may be described in the Examples or as otherwise known in the art.

As another examples, when the form of the immunomodulator is itself a particle or particle-like, such as a nanocrystalline immunomodulator, the load of immunomodulator is the amount of the immunomodulator in the particles or the like (weight/weight). In such cases, the load can approach 90%, 95%, 97%, 98%, 99% or more.

"Maximum dimension of a synthetic nanocarrier" means the largest dimension of a nanocarrier measured along any axis of the synthetic nanocarrier. "Minimum dimension of a synthetic nanocarrier" means the smallest dimension of a synthetic nanocarrier measured along any axis of the synthetic nanocarrier. For example, for a spheroidal synthetic nanocarrier, the maximum and minimum dimension of a synthetic nanocarrier would be substantially identical, and would be the size of its diameter. Similarly, for a cuboidal synthetic nanocarrier, the minimum dimension of a synthetic nanocarrier would be the smallest of its height, width or length, while the maximum dimension of a synthetic nanocarrier would be the largest of its height, width or length. In one case, a minimum dimension of at least 75%, preferably at least 80%, more preferably at least 90%, of the synthetic nanocarriers in a sample, based on the total number of synthetic nanocarriers in the sample, is equal to or greater than 100 nm. In one case, a maximum dimension of at least 75%, preferably at least 80%, more preferably at least 90%, of the synthetic nanocarriers in a sample, based on the total number of synthetic nanocarriers in the sample, is equal to or less than 5 µm. Preferably, a minimum dimension of at least 75%, preferably at least 80%, more preferably at least 90%, of the synthetic nanocarriers in a sample, based on the total number of synthetic nanocarriers in the sample, is greater than 110 nm, more preferably greater than 120 nm, more preferably greater than 130 nm, and more preferably still greater than 150 nm. Aspects ratios of the maximum and minimum dimensions of the synthetic nanocarriers may vary. For instance, aspect ratios of the maximum to minimum dimensions of the synthetic nanocarriers may vary from 1:1 to 1,000,000:1, preferably from 1:1 to 100,000:1, more preferably from 1:1 to 10,000:1, more preferably from 1:1 to 1000:1, still more preferably from 1:1 to 100:1, and yet more preferably from 1:1 to 10:1. Preferably, a maximum dimension of at least 75%, preferably at least 80%, more preferably at least 90%, of the synthetic nanocarriers in a sample, based on the total number of synthetic nanocarriers in the sample is equal to or less than 3 µm, more preferably equal to or less than 2 µm, more preferably equal to or less than 1 µm, more preferably equal to or less than 800 nm, more preferably equal to or less than 600 nm, and more preferably still equal to or less than 500 nm. In preferred cases, a minimum dimension of at least 75%, preferably at least 80%, more preferably at least 90%, of the synthetic nanocarriers in a sample, based on the total number of synthetic nanocarriers in the sample, is equal to or greater than 100 nm, more preferably equal to or greater than 120 nm, more preferably equal to or greater than 130 nm, more preferably equal to or greater than 140 nm, and more preferably still equal to or greater than 150 nm. Measurement of synthetic nanocarrier dimensions (e.g., diameter) is obtained by suspending the synthetic nanocarriers in a liquid (usually aqueous) media and using dynamic light scattering (DLS) (e.g. using a Brookhaven ZetaPALS instrument). For example, a suspension of synthetic nanocarriers can be diluted from an aqueous buffer into purified water to achieve a final synthetic nanocarrier suspension concentration of approximately 0.01 to 0.1 mg/mL. The diluted suspension may be prepared directly inside, or transferred to, a suitable cuvette for DLS analysis. The cuvette may then be placed in the DLS, allowed to equilibrate to the controlled temperature, and then scanned for sufficient time to acquire a stable and reproducible distribution based on appropriate inputs for viscosity of the medium and refractive indicies of the sample. The effective diameter, or mean of the distribution, is then reported. Determining the effective sizes of high aspect ratio, or non-spheroidal, synthetic nanocarriers may require augmentative techniques, such as electron microscopy, to obtain more accurate measurements. "Dimension" or "size" or "diameter" of synthetic nanocarriers means the mean of a particle size distribution, for example, obtained using dynamic light scattering.

"Pharmaceutically acceptable excipient" or "pharmaceutically acceptable carrier" means a pharmacologically inactive material used together with a pharmacologically active material to formulate the compositions. Pharmaceutically acceptable excipients comprise a variety of materials known in the art, including but not limited to saccharides (such as glucose and lactose), preservatives such as antimicrobial agents, reconstitution aids, colorants, saline (such as phosphate buffered saline), and buffers.

"Protocol" means a pattern of repeatedly administering antigen-specific immunotherapeutics to a subject. Protocols are made up of elements; thus a protocol comprises one or more elements. Such elements of the protocol can comprise dosing amounts, dosing frequency, routes of administration, dosing duration, dosing rates, intervals between dosing, and combinations of any of the foregoing. In some cases, a protocol may be used to administer one or more compositions to one or more test subjects. Immune responses in these test subjects can then be assessed to determine whether or not the protocol was effective in generating a desired or desired level of an immunologic effect. One or more of the elements may have been previously demonstrated in test subjects, such as non-human subjects, and then translated into human protocols. For example, dosing amounts demonstrated in non-human subjects can be scaled as an element of a human protocol using established techniques such as alimetric scaling or other scaling methods. Whether or not a protocol had a desired effect can be determined using any of the methods described herein or otherwise known in the art. For example, a population of cells may be obtained from a subject to which a recited composition and/or antigen-specific immunotherapeutic described herein has been repeatedly administered according to a specific protocol in order to determine whether or not specific immune cells, cytokines, antibodies, etc. were reduced, generated, activated, etc. Useful methods for detecting the presence and/or number of immune cells include, but are not limited to, flow cytometric methods (e.g., FACS) and immunohistochemistry methods. Antibodies and other binding agents for specific staining of immune cell markers, are commercially available. Such kits typically include staining reagents for multiple antigens that allow for FACS-based detection, separation and/or quantitation of a desired cell population from a heterogeneous population of cells. In some cases, the antigen-specific immunotherapeutic is repeatedly administered to another subject using all or substantially all of the elements of which the protocol is comprised.

"Protocol previously shown not to induce immunosuppression upon repeated administration" means a protocol wherein one or more of the elements of such protocol (up to and including the complete protocol) were demonstrated at a previous time not to result in immunosuppression during at least one point during, preferably the entirety of, repeated administration.

"Providing" means an action or set of actions that an individual performs that supply a needed item or set of items or methods for practicing of the present invention. The action or set of actions may be taken either directly oneself or indirectly.

"Providing a subject" is any action or set of actions that causes a clinician to come in contact with a subject and administer a composition described herein thereto or to perform a method described herein thereupon. Preferably, the subject is one who is in need of a tolerogenic immune response as described herein. The action or set of actions may be either directly oneself or indirectly. In one case of any one of the methods described herein, the method further comprises providing a subject.

"Repeated administration" or "repeatedly administer"or "repeatedly administering" and the like means boosting or extending the persistence of a previously established immune tolerance. These operations generally involve one administration or a short course of treatment at a time when the established tolerance is declining or at risk of declining. Repeated administration begins upon the next dose or doses of the antigen-specific immunotherapeutic administered following administration of an initial dose of an antigen-specific immunotherapeutic. The initial antigen-specific immunotherapeutic administered may be the same or different (in terms of composition, dosing, etc.) from the antigen-specific immunotherapeutic administered during repeated administration. Boosting is generally performed 2 weeks to 1 year, and preferably 1 to 6 months after an initial dose of the antigen-specific immunotherapeutic or a previous repeated administration. Also included are cases that involve regular repeated administrations on a schedule of administrations that occur semiweekly, weekly, biweekly, or on any other regular schedule.

"Subject" means animals, including warm blooded mammals such as humans and primates; avians; domestic household or farm animals such as cats, dogs, sheep, goats, cattle, horses and pigs; laboratory animals such as mice, rats and guinea pigs; fish; reptiles; zoo and wild animals.

"Synthetic nanocarrier(s)" means a discrete object that is not found in nature, and that possesses at least one dimension that is less than or equal to 5 microns in size. Albumin nanoparticles are generally included as synthetic nanocarriers, however in certain cases the synthetic nanocarriers do not comprise albumin nanoparticles. Synthetic nanocarriers may not comprise chitosan. In other cases, the synthetic nanocarriers are not lipid-based nanoparticles. In further cases, synthetic nanocarriers do not comprise a phospholipid.

A synthetic nanocarrier can be, but is not limited to, one or a plurality of lipid-based nanoparticles (also referred to herein as lipid nanoparticles, i.e., nanoparticles where the majority of the material that makes up their structure are lipids), polymeric nanoparticles, metallic nanoparticles, surfactant-based emulsions, dendrimers, buckyballs, nanowires, virus-like particles (i.e., particles that are primarily made up of viral structural proteins but that are not infectious or have low infectivity), peptide or protein-based particles (also referred to herein as protein particles, i.e., particles where the majority of the material that makes up their structure are peptides or proteins) (such as albumin nanoparticles) and/or nanoparticles that are developed using a combination of nanomaterials such as lipid-polymer nanoparticles. Synthetic nanocarriers may be a variety of different shapes, including but not limited to spheroidal, cuboidal, pyramidal, oblong, cylindrical, and toroidal. Synthetic nanocarriers comprise one or more surfaces. Exemplary synthetic nanocarriers that can be adapted for use in the practice of the present invention comprise: (1) the biodegradable nanoparticles disclosed in US Patent 5,543,158 to Gref et al., (2) the polymeric nanoparticles of Published US Patent Application 20060002852 to Saltzman et al., (3) the lithographically constructed nanoparticles of Published US Patent Application 20090028910 to DeSimone et al., (4) the disclosure of WO 2009/051837 to von Andrian et al., (5) the nanoparticles disclosed in Published US Patent Application 2008/0145441 to Penades et al., (6) the protein nanoparticles disclosed in Published US Patent Application 20090226525 to de los Rios et al., (7) the virus-like particles disclosed in published US Patent Application 20060222652 to Sebbel et al., (8) the nucleic acid coupled virus-like particles disclosed in published US Patent Application 20060251677 to Bachmann et al., (9) the virus-like particles disclosed in WO2010047839A1 or WO2009106999A2, (10) the nanoprecipitated nanoparticles disclosed in P. Paolicelli et al., "Surface-modified PLGA-based Nanoparticles that can Efficiently Associate and Deliver Virus-like Particles" Nanomedicine. 5(6): 843-853 (2010), (11) apoptotic cells, apoptotic bodies or the synthetic or semisynthetic mimics disclosed in U.S. Publication 2002/0086049, or (12) those of Look et al., Nanogel-based delivery of mycophenolic acid ameliorates systemic lupus erythematosus in mice" J. Clinical Investigation 123(4):1741-1749(2013). In some cases, synthetic nanocarriers may possess an aspect ratio greater than 1:1, 1:1.2, 1:1.5, 1:2, 1:3, 1:5, 1:7, or greater than 1:10.

Synthetic nanocarriers that have a minimum dimension of equal to or less than about 100 nm, preferably equal to or less than 100 nm, do not comprise a surface with hydroxyl groups that activate complement or alternatively comprise a surface that consists essentially of moieties that are not hydroxyl groups that activate complement. In a preferred case, synthetic nanocarriers that have a minimum dimension of equal to or less than about 100 nm, preferably equal to or less than 100 nm, do not comprise a surface that substantially activates complement or alternatively comprise a surface that consists essentially of moieties that do not substantially activate complement. In a more preferred case, synthetic nanocarriers that have a minimum dimension of equal to or less than about 100 nm, preferably equal to or less than 100 nm, do not comprise a surface that activates complement or alternatively comprise a surface that consists essentially of moieties that do not activate complement. In some cases, synthetic nanocarriers exclude virus-like particles. In some cases, synthetic nanocarriers may possess an aspect ratio greater than 1:1, 1:1.2, 1:1.5, 1:2, 1:3, 1:5, 1:7, or greater than 1:10.

"T cell antigen" means a CD4+ T-cell antigen or CD8+ cell antigen. "CD4+ T-cell antigen" means any antigen that is recognized by and triggers an immune response in a CD4+ T-cell e.g., an antigen that is specifically recognized by a T-cell receptor on a CD4+T cell via presentation of the antigen or portion thereof bound to a Class II major histocompatability complex molecule (MHC). "CD8+ T cell antigen" means any antigen that is recognized by and triggers an immune response in a CD8+ T-cell e.g., an antigen that is specifically recognized by a T-cell receptor on a CD8+T cell via presentation of the antigen or portion thereof bound to a Class I major histocompatability complex molecule (MHC). In some cases, an antigen that is a T cell antigen is also a B cell antigen. In other cases, the T cell antigen is not also a B cell antigen. T cell antigens generally are proteins or peptides.

A "therapeutic protein" refers to any protein or protein-based therapy that may be administered to a subject and have a therapeutic effect. Such therapies include protein replacement and protein supplementation therapies. Such therapies also include the administration of exogenous or foreign protein, antibody therapies, and cell or cell-based therapies. Therapeutic proteins include enzymes, enzyme cofactors, hormones, blood clotting factors, cytokines, growth factors, monoclonal antibodies and polyclonal antibodies. Examples of other therapeutic proteins are described elsewhere herein. Therapeutic proteins may be produced in, on or by cells and may be obtained from such cells or administered in the form of such cells. In some cases, the therapeutic protein is produced in, on or by mammalian cells, insect cells, yeast cells, bacteria cells, plant cells, transgenic animal cells, transgenic plant cells, etc. The therapeutic protein may be recombinantly produced in such cells. The therapeutic protein may also be produced in, on or by autologous cells that have been transfected, transduced or otherwise manipulated to express it. Alternatively, the therapeutic protein may be administered as a nucleic acid or by introducing a nucleic acid into a liposome, etc. Alternatively, the therapeutic protein may be obtained from such forms and administered as the therapeutic protein itself. Subjects, therefore, include any subject that has received, is receiving or will receive any of the foregoing.

"Undesired immune response" refers to any undesired immune response that results from exposure to an antigen, promotes or exacerbates a disease, disorder or condition described herein (or a symptom thereof), or is symptomatic of a disease, disorder or condition described herein. Such immune responses generally have a negative impact on a subject's health or is symptomatic of a negative impact on a subject's health. Undesired immune responses include antigen-specific antibody production, antigen-specific B cell proliferation and/or activity or antigen-specific CD4+ T cell proliferation and/or activity.

### C. TECHNICAL INFORMATION RELATING TO COMPOSITIONS

### Antigen-specific Immunotherapeutics

### Synthetic Nanocarriers

In some cases, the antigen-specific immunotherapeutics comprise synthetic nanocarrier compositions that comprise an immunomodulator and/or an antigen, together with related methods.

A wide variety of synthetic nanocarriers can be used. In some cases, synthetic nanocarriers are spheres or spheroids. In some cases, synthetic nanocarriers are flat or plate-shaped. In some cases, synthetic nanocarriers are cubes or cubic. In some cases, synthetic nanocarriers are ovals or ellipses. In some cases, synthetic nanocarriers are cylinders, cones, or pyramids.

In some cases, it is desirable to use a population of synthetic nanocarriers that is relatively uniform in terms of size, shape, and/or composition so that each synthetic nanocarrier has similar properties. For example, at least 80%, at least 90%, or at least 95% of the synthetic nanocarriers, based on the total number of synthetic nanocarriers, may have a minimum dimension or maximum dimension that falls within 5%, 10%, or 20% of the average diameter or average dimension of the synthetic nanocarriers.

Synthetic nanocarriers can be solid or hollow and can comprise one or more layers. In some cases, each layer has a unique composition and unique properties relative to the other layer(s). To give but one example, synthetic nanocarriers may have a core/shell structure, wherein the core is one layer (e.g. a polymeric core) and the shell is a second layer (e.g. a lipid bilayer or monolayer). Synthetic nanocarriers may comprise a plurality of different layers.

In some cases, synthetic nanocarriers may optionally comprise one or more lipids. In some cases, a synthetic nanocarrier may comprise a liposome. In some cases, a synthetic nanocarrier may comprise a lipid bilayer. In some cases, a synthetic nanocarrier may comprise a lipid monolayer. In some cases, a synthetic nanocarrier may comprise a micelle. In some cases, a synthetic nanocarrier may comprise a core comprising a polymeric matrix surrounded by a lipid layer (e.g., lipid bilayer, lipid monolayer, etc.). In some cases, a synthetic nanocarrier may comprise a non-polymeric core (e.g., metal particle, quantum dot, ceramic particle, bone particle, viral particle, proteins, nucleic acids, carbohydrates, etc.) surrounded by a lipid layer (e.g., lipid bilayer, lipid monolayer, etc.).

In other cases, synthetic nanocarriers may comprise metal particles, quantum dots, ceramic particles, etc. In some cases, a non-polymeric synthetic nanocarrier is an aggregate of non-polymeric components, such as an aggregate of metal atoms (e.g., gold atoms).

In some cases, synthetic nanocarriers may optionally comprise one or more amphiphilic entities. In some cases, an amphiphilic entity can promote the production of synthetic nanocarriers with increased stability, improved uniformity, or increased viscosity. In some cases, amphiphilic entities can be associated with the interior surface of a lipid membrane (e.g., lipid bilayer, lipid monolayer, etc.). Many amphiphilic entities known in the art are suitable for use in making synthetic nanocarriers. Such amphiphilic entities include, but are not limited to, phosphoglycerides; phosphatidylcholines; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohols such as polyethylene glycol (PEG); polyoxyethylene-9-lauryl ether; a surface active fatty acid, such as palmitic acid or oleic acid; fatty acids; fatty acid monoglycerides; fatty acid diglycerides; fatty acid amides; sorbitan trioleate (Span®85) glycocholate; sorbitan monolaurate (Span®20); polysorbate 20 (Tween®20); polysorbate 60 (Tween®60); polysorbate 65 (Tween®65); polysorbate 80 (Tween®80); polysorbate 85 (Tween®85); polyoxyethylene monostearate; surfactin; a poloxomer; a sorbitan fatty acid ester such as sorbitan trioleate; lecithin; lysolecithin; phosphatidylserine; phosphatidylinositol;sphingomyelin; phosphatidylethanolamine (cephalin); cardiolipin; phosphatidic acid; cerebrosides; dicetylphosphate; dipalmitoylphosphatidylglycerol; stearylamine; dodecylamine; hexadecyl-amine; acetyl palmitate; glycerol ricinoleate; hexadecyl sterate; isopropyl myristate; tyloxapol; poly(ethylene glycol)5000-phosphatidylethanolamine; poly(ethylene glycol)400-monostearate; phospholipids; synthetic and/or natural detergents having high surfactant properties; deoxycholates; cyclodextrins; chaotropic salts; ion pairing agents; and combinations thereof. An amphiphilic entity component may be a mixture of different amphiphilic entities. Those skilled in the art will recognize that this is an exemplary, not comprehensive, list of substances with surfactant activity. Any amphiphilic entity may be used in the production of synthetic nanocarriers.

In some cases, synthetic nanocarriers may optionally comprise one or more carbohydrates. Carbohydrates may be natural or synthetic. A carbohydrate may be a derivatized natural carbohydrate. In certain cases, a carbohydrate comprises monosaccharide or disaccharide, including but not limited to glucose, fructose, galactose, ribose, lactose, sucrose, maltose, trehalose, cellbiose, mannose, xylose, arabinose, glucoronic acid, galactoronic acid, mannuronic acid, glucosamine, galatosamine, and neuramic acid. In certain cases, a carbohydrate is a polysaccharide, including but not limited to pullulan, cellulose, microcrystalline cellulose, hydroxypropyl methylcellulose (HPMC), hydroxy cellulose (HC), methylcellulose (MC), dextran, cyclodextran, glycogen, hydroxyethylstarch, carageenan, glycon, amylose, chitosan, N,O-carboxylmethylchitosan, algin and alginic acid, starch, chitin, inulin, konjac, glucommannan, pustulan, heparin, hyaluronic acid, curdlan, and xanthan. In some cases, the synthetic nanocarriers do not comprise (or specifically exclude) carbohydrates, such as a polysaccharide. In certain cases, the carbohydrate may comprise a carbohydrate derivative such as a sugar alcohol, including but not limited to mannitol, sorbitol, xylitol, erythritol, maltitol, and lactitol.

In some cases, synthetic nanocarriers can comprise one or more polymers. In some cases, the synthetic nanocarriers comprise one or more polymers that is a non-methoxy-terminated, pluronic polymer. In some cases, at least 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, 97%, or 99% (weight/weight) of the polymers that make up the synthetic nanocarriers are non-methoxy-terminated, pluronic polymers. In some cases, all of the polymers that make up the synthetic nanocarriers are non-methoxy-terminated, pluronic polymers. In some cases, the synthetic nanocarriers comprise one or more polymers that is a non-methoxy-terminated polymer. In some cases, at least 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, 97%, or 99% (weight/weight) of the polymers that make up the synthetic nanocarriers are non-methoxy-terminated polymers. In some cases, all of the polymers that make up the synthetic nanocarriers are non-methoxy-terminated polymers. In some cases, the synthetic nanocarriers comprise one or more polymers that do not comprise pluronic polymer. In some cases, at least 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, 97%, or 99% (weight/weight) of the polymers that make up the synthetic nanocarriers do not comprise pluronic polymer. In some cases, all of the polymers that make up the synthetic nanocarriers do not comprise pluronic polymer. In some cases, such a polymer can be surrounded by a coating layer (e.g., liposome, lipid monolayer, micelle, etc.). In some cases, various elements of the synthetic nanocarriers can be coupled with the polymer.

The immunomodulators and/or antigens can be coupled to the synthetic nanocarriers by any of a number of methods. Generally, the coupling can be a result of bonding between the immunomodulators and/or antigens and the synthetic nanocarriers. This bonding can result in the immunomodulators and/or antigens being attached to the surface of the synthetic nanocarriers and/or contained within (encapsulated) the synthetic nanocarriers. In some cases, however, the immunomodulators and/or antigens are encapsulated by the synthetic nanocarriers as a result of the structure of the synthetic nanocarriers rather than bonding to the synthetic nanocarriers. In preferable cases, the synthetic nanocarrier comprises a polymer as described herein, and the immunomodulators and/or antigens are coupled to the polymer.

When coupling occurs as a result of bonding between the immunomodulators and/or antigens and synthetic nanocarriers, the coupling may occur via a coupling moiety. A coupling moiety can be any moiety through which an immunomodulator and/or antigen is bonded to a synthetic nanocarrier. Such moieties include covalent bonds, such as an amide bond or ester bond, as well as separate molecules that bond (covalently or non-covalently) the immunomodulator and/or antigen to the synthetic nanocarrier. Such molecules include linkers or polymers or a unit thereof. For example, the coupling moiety can comprise a charged polymer to which an immunomodulator and/or antigen electrostatically binds. As another example, the coupling moiety can comprise a polymer or unit thereof to which it is covalently bonded.

In preferred cases, the synthetic nanocarriers comprise a polymer as described herein. These synthetic nanocarriers can be completely polymeric or they can be a mix of polymers and other materials.

In some cases, the polymers of a synthetic nanocarrier associate to form a polymeric matrix. In some of these cases, a component, such as an immunomodulator or antigen, can be covalently associated with one or more polymers of the polymeric matrix. In some cases, covalent association is mediated by a linker. In some cases, a component can be noncovalently associated with one or more polymers of the polymeric matrix. For example, in some cases, a component can be encapsulated within, surrounded by, and/or dispersed throughout a polymeric matrix. Alternatively or additionally, a component can be associated with one or more polymers of a polymeric matrix by hydrophobic interactions, charge interactions, van der Waals forces, etc. A wide variety of polymers and methods for forming polymeric matrices therefrom are known conventionally.

Polymers may be natural or unnatural (synthetic) polymers. Polymers may be homopolymers or copolymers comprising two or more monomers. In terms of sequence, copolymers may be random, block, or comprise a combination of random and block sequences. Typically, polymers are organic polymers.

In some cases, the polymer comprises a polyester, polycarbonate, polyamide, or polyether, or unit thereof. In other cases, the polymer comprises poly(ethylene glycol) (PEG), polypropylene glycol, poly(lactic acid), poly(glycolic acid), poly(lactic-co-glycolic acid), or a polycaprolactone, or unit thereof. In some cases, it is preferred that the polymer is biodegradable. Therefore, in these cases, it is preferred that if the polymer comprises a polyether, such as poly(ethylene glycol) or polypropylene glycol or unit thereof, the polymer comprises a block-co-polymer of a polyether and a biodegradable polymer such that the polymer is biodegradable. In other cases, the polymer does not solely comprise a polyether or unit thereof, such as poly(ethylene glycol) or polypropylene glycol or unit thereof.

Other examples of polymers suitable for use in the present invention include, but are not limited to polyethylenes, polycarbonates (e.g. poly(1,3-dioxan-2one)), polyanhydrides (e.g. poly(sebacic anhydride)), polypropylfumerates, polyamides (e.g. polycaprolactam), polyacetals, polyethers, polyesters (e.g., polylactide, polyglycolide, polylactide-co-glycolide, polycaprolactone, polyhydroxyacid (e.g. poly((3-hydroxyalkanoate))), poly(orthoesters), polycyanoacrylates, polyvinyl alcohols, polyurethanes, polyphosphazenes, polyacrylates, polymethacrylates, polyureas, polystyrenes, and polyamines, polylysine, polylysine-PEG copolymers, and poly(ethyleneimine), poly(ethylene imine)-PEG copolymers.

In some cases, polymers include polymers which have been approved for use in humans by the U.S. Food and Drug Administration (FDA) under 21 C.F.R. § 177.2600, including but not limited to polyesters (e.g., polylactic acid, poly(lactic-co-glycolic acid), polycaprolactone, polyvalerolactone, poly(1,3-dioxan-2one)); polyanhydrides (e.g., poly(sebacic anhydride)); polyethers (e.g., polyethylene glycol); polyurethanes; polymethacrylates; polyacrylates; and polycyanoacrylates.

In some cases, polymers can be hydrophilic. For example, polymers may comprise anionic groups (e.g., phosphate group, sulphate group, carboxylate group); cationic groups (e.g., quaternary amine group); or polar groups (e.g., hydroxyl group, thiol group, amine group). In some cases, a synthetic nanocarrier comprising a hydrophilic polymeric matrix generates a hydrophilic environment within the synthetic nanocarrier. In some cases, polymers can be hydrophobic. In some cases, a synthetic nanocarrier comprising a hydrophobic polymeric matrix generates a hydrophobic environment within the synthetic nanocarrier. Selection of the hydrophilicity or hydrophobicity of the polymer may have an impact on the nature of materials that are incorporated (e.g. coupled) within the synthetic nanocarrier.

In some cases, polymers may be modified with one or more moieties and/or functional groups. A variety of moieties or functional groups can be used. In some cases, polymers may be modified with polyethylene glycol (PEG), with a carbohydrate, and/or with acyclic polyacetals derived from polysaccharides (Papisov, 2001, ACS Symposium Series, 786:301). The general teachings of US Patent No. 5543158 to Gref et al., or WO publication WO2009/051837 by Von Andrian et al. may be used.

In some cases, polymers may be modified with a lipid or fatty acid group. In some cases, a fatty acid group may be one or more of butyric, caproic, caprylic, capric, lauric, myristic, palmitic, stearic, arachidic, behenic, or lignoceric acid. In some cases, a fatty acid group may be one or more of palmitoleic, oleic, vaccenic, linoleic, alpha-linoleic, gamma-linoleic, arachidonic, gadoleic, arachidonic, eicosapentaenoic, docosahexaenoic, or erucic acid.

In some cases, polymers may be polyesters, including copolymers comprising lactic acid and glycolic acid units, such as poly(lactic acid-co-glycolic acid) and poly(lactide-co-glycolide), collectively referred to herein as "PLGA"; and homopolymers comprising glycolic acid units, referred to herein as "PGA," and lactic acid units, such as poly-L-lactic acid, poly-D-lactic acid, poly-D,L-lactic acid, poly-L-lactide, poly-D-lactide, and poly-D,L-lactide, collectively referred to herein as "PLA." In some cases, exemplary polyesters include, for example, polyhydroxy acids; PEG copolymers and copolymers of lactide and glycolide (e.g., PLA-PEG copolymers, PGA-PEG copolymers, PLGA-PEG copolymers, and derivatives thereof. In some cases, polyesters include, for example, poly(caprolactone), poly(caprolactone)-PEG copolymers, poly(L-lactide-co-L-lysine), poly(serine ester), poly(4-hydroxy-L-proline ester), poly[α-(4-aminobutyl)-L-glycolic acid], and derivatives thereof.

In some cases, a polymer may be PLGA. PLGA is a biocompatible and biodegradable co-polymer of lactic acid and glycolic acid, and various forms of PLGA are characterized by the ratio of lactic acid:glycolic acid. Lactic acid can be L-lactic acid, D-lactic acid, or D,L-lactic acid. The degradation rate of PLGA can be adjusted by altering the lactic acid:glycolic acid ratio. In some cases, PLGA is characterized by a lactic acid:glycolic acid ratio of approximately 85:15, approximately 75:25, approximately 60:40, approximately 50:50, approximately 40:60, approximately 25:75, or approximately 15:85.

In some cases, polymers may be one or more acrylic polymers. In certain cases, acrylic polymers include, for example, acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid anhydride), methyl methacrylate, polymethacrylate, poly(methyl methacrylate) copolymer, polyacrylamide, aminoalkyl methacrylate copolymer, glycidyl methacrylate copolymers, polycyanoacrylates, and combinations comprising one or more of the foregoing polymers. The acrylic polymer may comprise fully-polymerized copolymers of acrylic and methacrylic acid esters with a low content of quaternary ammonium groups.

In some cases, polymers can be cationic polymers. In general, cationic polymers are able to condense and/or protect negatively charged strands of nucleic acids (e.g. DNA, or derivatives thereof). Amine-containing polymers such as poly(lysine) (Zauner et al., 1998, Adv. Drug Del. Rev., 30:97; and Kabanov et al., 1995, Bioconjugate Chem., 6:7), poly(ethylene imine) (PEI; Boussif et al., 1995, Proc. Natl. Acad. Sci., USA, 1995, 92:7297), and poly(amidoamine) dendrimers (Kukowska-Latallo et al., 1996, Proc. Natl. Acad. Sci., USA, 93:4897; Tang et al., 1996, Bioconjugate Chem., 7:703; and Haensler et al., 1993, Bioconjugate Chem., 4:372) are positively-charged at physiological pH, form ion pairs with nucleic acids, and mediate transfection in a variety of cell lines. In some cases, the synthetic nanocarriers may not comprise (or may exclude) cationic polymers.

In some cases, polymers can be degradable polyesters bearing cationic side chains (Putnam et al., 1999, Macromolecules, 32:3658; Barrera et al., 1993, J. Am. Chem. Soc., 115:11010; Kwon et al., 1989, Macromolecules, 22:3250; Lim et al., 1999, J. Am. Chem. Soc., 121:5633; and Zhou et al., 1990, Macromolecules, 23:3399). Examples of these polyesters include poly(L-lactide-co-L-lysine) (Barrera et al., 1993, J. Am. Chem. Soc., 115:11010), poly(serine ester) (Zhou et al., 1990, Macromolecules, 23:3399), poly(4-hydroxy-L-proline ester) (Putnam et al., 1999, Macromolecules, 32:3658; and Lim et al., 1999, J. Am. Chem. Soc., 121:5633), and poly(4-hydroxy-L-proline ester) (Putnam et al., 1999, Macromolecules, 32:3658; and Lim et al., 1999, J. Am. Chem. Soc., 121:5633).

The properties of these and other polymers and methods for preparing them are well known in the art (see, for example, U.S. Patents 6,123,727; 5,804,178; 5,770,417; 5,736,372; 5,716,404; 6,095,148; 5,837,752; 5,902,599; 5,696,175; 5,514,378; 5,512,600; 5,399,665; 5,019,379; 5,010,167; 4,806,621; 4,638,045; and 4,946,929; Wang et al., 2001, J. Am. Chem. Soc., 123:9480; Lim et al., 2001, J. Am. Chem. Soc., 123:2460; Langer, 2000, Acc. Chem. Res., 33:94; Langer, 1999, J. Control. Release, 62:7; and Uhrich et al., 1999, Chem. Rev., 99:3181). More generally, a variety of methods for synthesizing certain suitable polymers are described in Concise Encyclopedia of Polymer Science and Polymeric Amines and Ammonium Salts, Ed. by Goethals, Pergamon Press, 1980; Principles of Polymerization by Odian, John Wiley & Sons, Fourth Edition, 2004; Contemporary Polymer Chemistry by Allcock et al., Prentice-Hall, 1981; Deming et al., 1997, Nature, 390:386; and in U.S. Patents 6,506,577, 6,632,922, 6,686,446, and 6,818,732.

In some cases, polymers can be linear or branched polymers. In some cases, polymers can be dendrimers. In some cases, polymers can be substantially cross-linked to one another. In some cases, polymers can be substantially free of cross-links. In some cases, polymers can be used without undergoing a cross-linking step. It is further to be understood that synthetic nanocarriers may comprise block copolymers, graft copolymers, blends, mixtures, and/or adducts of any of the foregoing and other polymers. Those skilled in the art will recognize that the polymers listed herein represent an exemplary, not comprehensive, list of polymers that can be of use.

In some cases, synthetic nanocarriers do not comprise a polymeric component. In some cases, synthetic nanocarriers may comprise metal particles, quantum dots, ceramic particles, etc. In some cases, a non-polymeric synthetic nanocarrier is an aggregate of non-polymeric components, such as an aggregate of metal atoms (e.g., gold atoms).

Compositions may comprise synthetic nanocarriers in combination with pharmaceutically acceptable excipients, such as preservatives, buffers, saline, or phosphate buffered saline. The compositions may be made using conventional pharmaceutical manufacturing and compounding techniques to arrive at useful dosage forms. In one case, synthetic nanocarriers are suspended in sterile saline solution for injection together with a preservative.

In some cases, when preparing synthetic nanocarriers as carriers, methods for coupling components to the synthetic nanocarriers may be useful. If the component is a small molecule it may be of advantage to attach the component to a polymer prior to the assembly of the synthetic nanocarriers. In some cases, it may also be an advantage to prepare the synthetic nanocarriers with surface groups that are used to couple the component to the synthetic nanocarrier through the use of these surface groups rather than attaching the component to a polymer and then using this polymer conjugate in the construction of synthetic nanocarriers.

In certain cases, the coupling can be a covalent linker. In some cases, peptides can be covalently coupled to the external surface via a 1,2,3-triazole linker formed by the 1,3-dipolar cycloaddition reaction of azido groups on the surface of the nanocarrier with antigen or immunomodulator containing an alkyne group or by the 1,3-dipolar cycloaddition reaction of alkynes on the surface of the nanocarrier with antigens or immunomodulators containing an azido group. Such cycloaddition reactions are preferably performed in the presence of a Cu(I) catalyst along with a suitable Cu(I)-ligand and a reducing agent to reduce Cu(II) compound to catalytic active Cu(I) compound. This Cu(I)-catalyzed azide-alkyne cycloaddition (CuAAC) can also be referred as the click reaction.

Additionally, the covalent coupling may comprise a covalent linker that comprises an amide linker, a disulfide linker, a thioether linker, a hydrazone linker, a hydrazide linker, an imine or oxime linker, an urea or thiourea linker, an amidine linker, an amine linker, and a sulfonamide linker.

An amide linker is formed via an amide bond between an amine on one component such as an antigen or immunomodulator with the carboxylic acid group of a second component such as the nanocarrier. The amide bond in the linker can be made using any of the conventional amide bond forming reactions with suitably protected amino acids and activated carboxylic acid such N-hydroxysuccinimide-activated ester.

A disulfide linker is made via the formation of a disulfide (S-S) bond between two sulfur atoms of the form, for instance, of R1-S-S-R2. A disulfide bond can be formed by thiol exchange of a component containing thiol/mercaptan group(-SH) with another activated thiol group on a polymer or nanocarrier or a nanocarrier containing thiol/mercaptan groups with a component containing activated thiol group.

A triazole linker, specifically a 1,2,3-triazole of the form 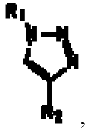 wherein R1 and R2 may be any chemical entities, is made by the 1,3-dipolar cycloaddition reaction of an azide attached to a first component such as the nanocarrier with a terminal alkyne attached to a second component such as the immunomodulator or antigen. The 1,3-dipolar cycloaddition reaction is performed with or without a catalyst, preferably with Cu(I)-catalyst, which links the two components through a 1,2,3-triazole function. This chemistry is described in detail by Sharpless et al., Angew. Chem. Int. Ed. 41(14), 2596, (2002) and Meldal, et al, Chem. Rev., 2008, 108(8), 2952-3015 and is often referred to as a "click" reaction or CuAAC.

In some cases, a polymer containing an azide or alkyne group, terminal to the polymer chain is prepared. This polymer is then used to prepare a synthetic nanocarrier in such a manner that a plurality of the alkyne or azide groups are positioned on the surface of that nanocarrier. Alternatively, the synthetic nanocarrier can be prepared by another route, and subsequently functionalized with alkyne or azide groups. The component is prepared with the presence of either an alkyne (if the polymer contains an azide) or an azide (if the polymer contains an alkyne) group. The component is then allowed to react with the nanocarrier via the 1,3-dipolar cycloaddition reaction with or without a catalyst which covalently couples the component to the particle through the 1,4-disubstituted 1,2,3-triazole linker.

A thioether linker is made by the formation of a sulfur-carbon (thioether) bond in the form, for instance, of R1-S-R2. Thioether can be made by either alkylation of a thiol/mercaptan (-SH) group on one component with an alkylating group such as halide or epoxide on a second component. Thioether linkers can also be formed by Michael addition of a thiol/mercaptan group on one component to an electron-deficient alkene group on a second component containing a maleimide group or vinyl sulfone group as the Michael acceptor. In another way, thioether linkers can be prepared by the radical thiol-ene reaction of a thiol/mercaptan group on one component with an alkene group on a second component.

A hydrazone linker is made by the reaction of a hydrazide group on one component with an aldehyde/ketone group on the second component.

A hydrazide linker is formed by the reaction of a hydrazine group on one component with a carboxylic acid group on the second component. Such reaction is generally performed using chemistry similar to the formation of amide bond where the carboxylic acid is activated with an activating reagent.

An imine or oxime linker is formed by the reaction of an amine or N-alkoxyamine (or aminooxy) group on one component with an aldehyde or ketone group on the second component.

An urea or thiourea linker is prepared by the reaction of an amine group on one component with an isocyanate or thioisocyanate group on the second component.

An amidine linker is prepared by the reaction of an amine group on one component with an imidoester group on the second component.

An amine linker is made by the alkylation reaction of an amine group on one component with an alkylating group such as halide, epoxide, or sulfonate ester group on the second component. Alternatively, an amine linker can also be made by reductive amination of an amine group on one component with an aldehyde or ketone group on the second component with a suitable reducing reagent such as sodium cyanoborohydride or sodium triacetoxyborohydride.

A sulfonamide linker is made by the reaction of an amine group on one component with a sulfonyl halide (such as sulfonyl chloride) group on the second component.

A sulfone linker is made by Michael addition of a nucleophile to a vinyl sulfone. Either the vinyl sulfone or the nucleophile may be on the surface of the nanocarrier or attached to a component.

The component can also be conjugated to the nanocarrier via non-covalent conjugation methods. For example, a negative charged antigen or immunomodulator can be conjugated to a positive charged nanocarrier through electrostatic adsorption. A component containing a metal ligand can also be conjugated to a nanocarrier containing a metal complex via a metal-ligand complex.

In some cases, the component can be attached to a polymer, for example polylactic acid-block-polyethylene glycol, prior to the assembly of the synthetic nanocarrier or the synthetic nanocarrier can be formed with reactive or activatible groups on its surface. In the latter case, the component may be prepared with a group which is compatible with the attachment chemistry that is presented by the synthetic nanocarriers' surface. In other cases, a peptide component can be attached to VLPs or liposomes using a suitable linker. A linker is a compound or reagent that capable of coupling two molecules together. In one case, the linker can be a homobifuntional or heterobifunctional reagent as described in Hermanson 2008. For example, an VLP or liposome synthetic nanocarrier containing a carboxylic group on the surface can be treated with a homobifunctional linker, adipic dihydrazide (ADH), in the presence of EDC to form the corresponding synthetic nanocarrier with the ADH linker. The resulting ADH linked synthetic nanocarrier is then conjugated with a peptide component containing an acid group via the other end of the ADH linker on NC to produce the corresponding VLP or liposome peptide conjugate.

For detailed descriptions of available conjugation methods, see Hermanson G T "Bioconjugate Techniques", 2nd Edition Published by Academic Press, Inc., 2008. In addition to covalent attachment the component can be coupled by adsorption to a pre-formed synthetic nanocarrier or it can be coupled by encapsulation during the formation of the synthetic nanocarrier.

### Modified Antigens

In some cases, any one of the recited compositions and/or the recited antigen-specific immunotherapeutics can comprise a modified antigen, wherein the modification can serve a variety of purposes, including but not limited to increased circulation stability (such as pegylation of protein or peptide antigens), reduced sensitivity to peptidase degradation (such as substitution of non-natural amino acids for natural amino acids), and to enhance tolergenic performance (such as attachment to erythrocytes).

In a preferred case, the modified antigen comprises a fusion protein that comprises an antigen of interest fused with a binding moiety that binds erythrocytes. An example of such a binding moiety comprises a synthetic 12-aa peptide (ERY1) described in the literature as H₂N-WMVLPWLPGTLDGGSGCRGCONH₂ (SEQ ID NO: 1), which includes a 12-mer sequence described in the literature as a mouse glycophorin-A binder. The GGSG region was described to serve as a linker to the cysteine residue used for conjugation, and the the flanking arginine residue was described to serve to lower the pKa, and thus to increase the reactivity of the cysteine residue. In another case, a fusion protein can be generated that comprises the antigen of interest fused with a binding moiety such as a murine glycophorin A-binding moiety or an equivalent such moiety for other species (e.g. humans). In a specific case, murine glycophorin A-binding TER-119 Ab, or fragments thereof (such as a TER-119 scFv, can be fused with the antigen of interest. See, generally, S. Kontos et al., "Engineering antigens for in situ erythrocyte binding induces T-cell deletion" Proc Natl Acad Sci USA. 2013 Jan 2;110(1):E60-8 ("Kontsos"). Additional erythrocyte binding moieties can be generated using the phage display or mAb/mAb fragment approaches generally disclosed in the Kontos article and in the relevant literature.

Modified antigens can be formulated in a variety of ways, for administration using a variety of routes. Appropriate formulation approaches, and useful routes, are disclosed elsewhere herein and can be applied to compositions and/or antigen-specific imunnotherapeutics according to the present disclosure.

### Expressed Antigen:

In some cases, the recited compositions and/or the recited antigen-specific immunotherapeutics can comprise an expressed antigen, wherein the expressed antigen is expressed following delivery of a genetic construct, preferably a non-highly immunogenic genetic construct. Examples of such genetic constructs are known in the art, and include, but is not limited to, direct injection, liposomal, cationic lipid; or condensed DNA/RNA particles, or gene gun delivery of: various constructs comprising DNA or RNA; plasmids; or naked DNA or RNA (including cDNA, messenger RNA, modified messenger RNA, and forms of RNAi). See generally J. R. Ohlfest et al., "Phenotypic correction and long-term expression of factor VIII in hemophilic mice by immunotolerization and nonviral gene transfer using the Sleeping Beauty transposon system" Blood 2005;105:2691-2698; A Tautzenberger et al., "Nanoparticles and their potential for application in bone" Int'l. J. of Nanomedicine 2012:7 4545-4557. Modified messenger RNAs, including direct injection thereof, are disclosed in Published US Patent application 2013/0115272 to de Fougerolles et al. and in Published US Patent application 2012/0251618 to Schrum et al. Any of the proteins listed elsewhere herein, or known generally in the art may be considered for delivery in the context of an expressed antigen.

Expressed antigens can be formulated in a variety of ways, for administration using a variety of routes. Appropriate formulation approaches, and useful routes, are disclosed elsewhere herein and can be applied to compositions and/or antigen-specific imunnotherapeutics according to the present disclosure.

### Antigens

Antigens useful in the practice of the present invention can be selected from a broad range of antigens, including exogenous and endogenous antigens.

### Exogenous Antigens

Exogenous antigens, as noted elsewhere herein, can comprise therapeutic proteins, modified antigens, and expressed antigens.

Therapeutic proteins can comprise any of the therapeutic proteins, or fragments or derivatives thereof, described herein. Therapeutic proteins include, but are not limited to, infusible therapeutic proteins, enzymes, enzyme cofactors, hormones, blood clotting factors, cytokines and interferons, growth factors, monoclonal antibodies, and polyclonal antibodies, and proteins associated with Pompe's disease (e.g., alglucosidase alfa, rhGAA (e.g., Myozyme and Lumizyme (Genzyme)) (e.g., that are administered to a subject as a replacement therapy). Therapeutic proteins also include proteins involved in the blood coagulation cascade. Therapeutic proteins include, but are not limited to, Factor VIII, Factor VII, Factor IX, Factor V, von Willebrand Factor, von Heldebrant Factor, tissue plasminogen activator, insulin, growth hormone, erythropoietin alfa, VEGF, thrombopoietin, lysozyme, and antithrombin. Therapeutic proteins also include adipokines, such as leptin and adiponectin. Other examples of therapeutic proteins are as described below and elsewhere herein. Also included are fragments or derivatives of any of the therapeutic proteins described as the antigen.

Examples of therapeutic proteins used in enzyme replacement therapy of subjects having a lysosomal storage disorder include, but are not limited to, imiglucerase for the treatment of Gaucher's disease (e.g., CEREZYME™), a-galactosidase A (a-gal A) for the treatment of Fabry disease (e.g., agalsidase beta, FABRYZYME™), acid a-glucosidase (GAA) for the treatment of Pompe disease (e.g., alglucosidase alfa, LUMIZYME™, MYOZYME™), arylsulfatase B for the treatment of Mucopolysaccharidoses (e.g., laronidase, ALDURAZYME™, idursulfase, ELAPRASE™, arylsulfatase B, AGLAZYME™).

Examples of enzymes include oxidoreductases, transferases, hydrolases, lyases, isomerases, and ligases.

Examples of hormones include Melatonin (N-acetyl-5-methoxytryptamine), Serotonin, Thyroxine (or tetraiodothyronine) (a thyroid hormone), Triiodothyronine (a thyroid hormone), Epinephrine (or adrenaline), Norepinephrine (or noradrenaline), Dopamine (or prolactin inhibiting hormone), Antimullerian hormone (or mullerian inhibiting factor or hormone), Adiponectin, Adrenocorticotropic hormone (or corticotropin), Angiotensinogen and angiotensin, Antidiuretic hormone (or vasopressin, arginine vasopressin), Atrial-natriuretic peptide (or atriopeptin), Calcitonin, Cholecystokinin, Corticotropin-releasing hormone, Erythropoietin, Follicle-stimulating hormone, Gastrin, Ghrelin, Glucagon, Glucagon-like peptide (GLP-1), GIP, Gonadotropin-releasing hormone, Growth hormone-releasing hormone, Human chorionic gonadotropin, Human placental lactogen, Growth hormone, Inhibin, Insulin, Insulin-like growth factor (or somatomedin), Leptin, Luteinizing hormone, Melanocyte stimulating hormone, Orexin, Oxytocin, Parathyroid hormone, Prolactin, Relaxin, Secretin, Somatostatin, Thrombopoietin, Thyroid-stimulating hormone (or thyrotropin), Thyrotropin-releasing hormone, Cortisol, Aldosterone, Testosterone, Dehydroepiandrosterone, Androstenedione, Dihydrotestosterone, Estradiol, Estrone, Estriol, Progesterone, Calcitriol (1,25-dihydroxyvitamin D3), Calcidiol (25-hydroxyvitamin D3), Prostaglandins, Leukotrienes, Prostacyclin, Thromboxane, Prolactin releasing hormone, Lipotropin, Brain natriuretic peptide, Neuropeptide Y, Histamine, Endothelin, Pancreatic polypeptide, Renin, and Enkephalin.

Examples of blood and blood coagulation factors include Factor I (fibrinogen), Factor II (prothrombin), tissue factor, Factor V (proaccelerin, labile factor), Factor VII (stable factor, proconvertin), Factor VIII (antihemophilic globulin), Factor IX (Christmas factor or plasma thromboplastin component), Factor X (Stuart-Prower factor), Factor Xa, Factor XI, Factor XII (Hageman factor), Factor XIII (fibrin-stabilizing factor), von Willebrand factor, prekallikrein (Fletcher factor), high-molecular weight kininogen (HMWK) (Fitzgerald factor), fibronectin, fibrin, thrombin, antithrombin III, heparin cofactor II, protein C, protein S, protein Z, protein Z-related protease inhibitot (ZPI), plasminogen, alpha 2-antiplasmin, tissue plasminogen activator (tPA), urokinase, plasminogen activator inhibitor-1 (PAI1), plasminogen activator inhibitor-2 (PAI2), cancer procoagulant, and epoetin alfa (Epogen, Procrit).

Examples of cytokines include lymphokines, interleukins, and chemokines, type 1 cytokines, such as IFN-γ, TGF-β, and type 2 cytokines, such as IL-4, IL-10, and IL-13.

Examples of growth factors include Adrenomedullin (AM), Angiopoietin (Ang), Autocrine motility factor, Bone morphogenetic proteins (BMPs), Brain-derived neurotrophic factor (BDNF), Epidermal growth factor (EGF), Erythropoietin (EPO), Fibroblast growth factor (FGF), Glial cell line-derived neurotrophic factor (GDNF), Granulocyte colony-stimulating factor (G-CSF), Granulocyte macrophage colony-stimulating factor (GM-CSF), Growth differentiation factor-9 (GDF9), Hepatocyte growth factor (HGF), Hepatoma-derived growth factor (HDGF), Insulin-like growth factor (IGF), Migration-stimulating factor, Myostatin (GDF-8), Nerve growth factor (NGF) and other neurotrophins, Platelet-derived growth factor (PDGF), Thrombopoietin (TPO), Transforming growth factor alpha(TGF-α), Transforming growth factor beta(TGF-β), Tumour_necrosis factor-alpha(TNF-α), Vascular endothelial growth factor (VEGF), Wnt Signaling Pathway, placental growth factor (PlGF), [(Foetal Bovine Somatotrophin)] (FBS), IL-1, IL-2, IL-3, IL-4, IL-5, IL-6, and IL-7.

Examples of monoclonal antibodies include Abagovomab, Abciximab, Adalimumab, Adecatumumab, Afelimomab, Afutuzumab, Alacizumab pegol, ALD, Alemtuzumab, Altumomab pentetate, Anatumomab mafenatox, Anrukinzumab, Anti-thymocyte globin, Apolizumab, Arcitumomab, Aselizumab, Atlizumab (tocilizumab), Atorolimumab, Bapineuzumab, Basiliximab, Bavituximab, Bectumomab, Belimumab, Benralizumab, Bertilimumab, Besilesomab, Bevacizumab, Biciromab, Bivatuzumab mertansine, Blinatumomab, Brentuximab vedotin, Briakinumab, Canakinumab, Cantuzumab mertansine, Capromab pendetide, Catumaxomab, Cedelizumab, Certolizumab pegol, Cetuximab, Citatuzumab bogatox, Cixutumumab, Clenoliximab, Clivatuzumab tetraxetan, Conatumumab, Dacetuzumab, Daclizumab, Daratumumab, Denosumab, Detumomab, Dorlimomab aritox, Dorlixizumab, Ecromeximab, Eculizumab, Edobacomab, Edrecolomab, Efalizumab, Efungumab, Elotuzumab, Elsilimomab, Enlimomab pegol, Epitumomab cituxetan, Epratuzumab, Erlizumab, Ertumaxomab, Etaracizumab, Exbivirumab, Fanolesomab, Faralimomab, Farletuzumab, Felvizumab, Fezakinumab, Figitumumab, Fontolizumab , Foravirumab, Fresolimumab, Galiximab, Gantenerumab, Gavilimomab, Gemtuzumab ozogamicin, GC1008, Girentuximab, Glembatumumab vedotin, Golimumab, Gomiliximab, Ibalizumab, Ibritumomab tiuxetan, Igovomab, Imciromab, Infliximab, Intetumumab, Inolimomab, Inotuzumab ozogamicin, Ipilimumab, Iratumumab, Keliximab, Labetuzumab, Lebrikizumab, Lemalesomab, Lerdelimumab, Lexatumumab, Libivirumab, Lintuzumab, Lorvotuzumab mertansine, Lucatumumab, Lumiliximab, Mapatumumab, Maslimomab, Matuzumab, Mepolizumab, Metelimumab, Milatuzumab, Minretumomab, Mitumomab, Morolimumab, Motavizumab, Muromonab-CD3, Nacolomab tafenatox, Naptumomab estafenatox, Natalizumab, Nebacumab, Necitumumab, Nerelimomab, Nimotuzumab, Nofetumomab merpentan, Ocrelizumab, Odulimomab, Ofatumumab, Olaratumab, Omalizumab, Oportuzumab monatox, Oregovomab, Otelixizumab, Pagibaximab, Palivizumab, Panitumumab, Panobacumab, Pascolizumab, Pemtumomab, Pertuzumab, Pexelizumab, Pintumomab, Priliximab, Pritumumab, Rafivirumab, Ramucirumab, Ranibizumab, Raxibacumab, Regavirumab Reslizumab, Rilotumumab, Rituximab, Robatumumab, Rontalizumab, Rovelizumab, Ruplizumab, Satumomab pendetide, Sevirumab, Sibrotuzumab, Sifalimumab, Siltuximab, Siplizumab, Solanezumab, Sonepcizumab, Sontuzumab, Stamulumab, Sulesomab, Tacatuzumab tetraxetan, Tadocizumab, Talizumab, Tanezumab, Taplitumomab paptox, Tefibazumab, Telimomab aritox, Tenatumomab, Teneliximab, Teplizumab, Ticilimumab (tremelimumab), Tigatuzumab, Tocilizumab (atlizumab), Toralizumab, Tositumomab, Trastuzumab, Tremelimumab, Tucotuzumab celmoleukin, Tuvirumab, Urtoxazumab, Ustekinumab, Vapaliximab, Vedolizumab, Veltuzumab, Vepalimomab, Visilizumab, Volociximab, Votumumab, Zalutumumab, Zanolimumab, Ziralimumab, and Zolimomab aritox.

Examples of infusion therapy or injectable therapeutic proteins include, for example, Tocilizumab (Roche/Actemra®), alpha-1 antitrypsin (Kamada/AAT), Hematide® (Affymax and Takeda, synthetic peptide), albinterferon alfa-2b (Novartis/Zalbin™), Rhucin® (Pharming Group, C1 inhibitor replacement therapy), tesamorelin (Theratechnologies/Egrifta, synthetic growth hormone-releasing factor), ocrelizumab (Genentech, Roche and Biogen), belimumab (GlaxoSmithKline/Benlysta®), pegloticase (Savient Pharmaceuticals/Krystexxa™), pegsiticase, taliglucerase alfa (Protalix/Uplyso), agalsidase alfa (Shire/Replagal®), velaglucerase alfa (Shire).

Additional therapeutic proteins useful in accordance to aspects of this invention will be apparent to those of skill in the art, and the invention is not limited in this respect.

Additional exogenous antigens may comprise modified antigens or expressed antigens.

Modified antigens, such as fusion constructs between an antigen of interest and a moiety that targets erythrocytes, have been described elsewhere herein and can be useful in the practice of the present invention.

Expressed antigens, such as antigens generated by administration of non- or poorly-immunogenic gene vectors, plasmid DNA, nucleic acids (e.g. DNA or RNA), or modified nucleic acids, have been described elsewhere herein and can be useful in the practice of the present invention.

### Endogenous Antigens

Some compositions described herein comprise antigens that are endogenous antigens. Endogenous antigens comprise autoantigens, such as those found in autoimmune diseases. Autoantigens include, but are not limited to, those found in Anklosing spondylitis; bulous pemiphigous; rheumatoid arthritis; multiple sclerosis; diabetes, including but not limited to insulin-dependent diabetes mellitus, diabetes mellitus, juvenile diabetes, spontaneous autoimmune diabetes, immune-mediated or Type I diabetes mellitus; excema; inflammatory bowel disease (e.g., Crohn's disease or ulcerative colitis) such as autoimmune inflammatory bowel disease; lupus or systemic lupus erythematosus; multiple sclerosis; primary biliary cirrhosis; psoriasis; sarcoidosis; systemic sclerosis; scleroderma; thyroiditis; autoimmune thyroid disease; Hashimoto's thyroiditis; thyrotoxicosis; alopecia areata; Grave's disease; Guillain-Barre syndrome; celiac disease; Sjögren's syndrome; rheumatic fever; gastritis autoimmune atrophic gastritis; autoimmune hepatitis; insulitis; oophoritis; orchitis; uveitis; phacogenic uveitis; myasthenia gravis; primary myxoedema; pernicious anemia; primary sclerosing cholangitis; autoimmune haemolytic anemia; Addison's disease; scleroderma; Goodpasture's syndrome; nephritis, for example, glomerulonephritis; psoriasis; pemphigus vulgaris; pemphigoid; sympathetic opthalmia; idiopathic thrombocylopenic purpura; idiopathic feucopenia; Wegener's granulomatosis and poly/dermatomyositis.

Some additional exemplary autoimmune diseases, associated autoantigens, and autoantibodies, which are contemplated, are described in Table 1 below:

| Autoantibody Type | Autoantibody | Autoantigen | Autoimmune disease or disorder |
|---|---|---|---|
| Antinuclear antibodies | Anti-SSA/Ro autoantibodies | ribonucleoproteins | Systemic lupus erythematosus, neonatal heart block, primary Sjogren's syndrome |
| | Anti-La/SS-B autoantibodies | ribonucleoproteins | Primary Sjögren's syndrome |
| | Anti-centromere antibodies | centromere | CREST syndrome |
| | Anti-neuronal nuclear antibody-2 | Ri[disambiguation needed] | Opsoclonus |
| | Anti-dsDNA | double-stranded DNA | SLE |
| | Anti-Jo1 | histidine-tRNA ligase | Inflammatory myopathy |
| | Anti-Smith | snRNP core proteins | SLE |
| | Anti-topoisomerase antibodies | Type I topoisomerase | Systemic sclerosis (anti-Scl-70 antibodies) |
| | Anti-histone antibodies | histones | SLE and Drug-induced LE[2] |
| | Anti-p62 antibodies [3] | nucleoporin 62 | Primary biliary cirrhosis [3] [4] [5] |
| | Anti-sp100 antibodies [4] | Sp100 nuclear antigen | |
| | Anti-glycoprotein-210 antibodies[5] | nucleoporin 210kDa | |
| Anti-trans glutaminase antibodies | Anti-tTG | | Coeliac disease |
| | Anti-eTG | | Dermatitis herpetiformis |
| Anti-ganglioside antibodies | | ganglioside GQ1B | Miller-Fisher Syndrome |
| | | ganglioside GD3 | Acute motor axonal neuropathy (AMAN) |
| | | ganglioside GM1 | Multifocal motor neuropathy with conduction block (MMN) |
| Anti-actin antibodies | | actin | Coeliac disease anti-actin antibodies correlated with the level of intestinal damage [6][7] |
| Liver kidney microsomal type 1 antibody | | | Autoimmune hepatitis. [8] |
| Lupus anticoagulant | Anti-thrombin antibodies | thrombin | Systemic lupus erythematosus |
| Anti-neutrophil cytoplasmic antibody | | phospholipid | Antiphospholipid syndrome |
| | c-ANCA | proteins in neutrophil cytoplasm | Wegener's granulomatosis |
| | p-ANCA | neutrophil perinuclear | Microscopic polyangiitis, Churg-Strauss syndrome, systemic vasculitides (nonspecific) |
| Rheumatoid factor | | IgG | Rheumatoid arthritis |
| Anti-smooth muscle antibody | | smooth muscle | Chronic autoimmune hepatitis |
| Anti-mitochondrial antibody | | mitochondria | Primary biliary cirrhosis[9] |
| Anti-SRP | | signal recognition particle | Polymyositis[10] |
| | | exosome complex | Scleromyositis |
| | | nicotinic acetylcholine receptor | Myasthenia gravis |
| | | muscle-specific kinase (MUSK) | Myasthenia gravis |
| Anti-VGCC | | voltage-gated calcium channel (P/Q-type) | Lambert-Eaton myasthenic syndrome |
| | | thyroid peroxidase (microsomal) | Hashimoto's thyroiditis |
| | | TSH receptor | Graves' disease |
| | | Hu | Paraneoplastic cerebellar syndrome |
| | | Yo (cerebellar Purkinje Cells) | Paraneoplastic cerebellar syndrome |
| | | amphiphysin | Stiff person syndrome, paraneoplastic cerebellar syndrome |
| Anti-VGKC | | voltage-gated potassium channel (VGKC) | Limbic encephalitis, Isaac's Syndrome (autoimmune neuromyotonia) |
| | | basal ganglia neurons | Sydenham's chorea, paediatric autoimmune neuropsychiatric disease associated with Streptococcus (PANDAS) |
| | | N-methyl-D-aspartate receptor (NMDA) | Encephalitis |
| | | glutamic acid decarboxylase (GAD) | Diabetes mellitus type 1, stiff person syndrome |
| | | aquaporin-4 | Neuromyelitis optica (Devic's syndrome) |

Endogenous antigens may also include those associated with transplanted tissue, such as solid organ transplant or bone marrow transplant. Graft versus host disease (GVHD) is a complication that can occur after a pluripotent cell (e.g., stem cell) or bone marrow transplant in which the newly transplanted material results in an attack on the transplant recipient's body. In some instances, GVHD takes place after a blood transfusion.

Additional endogenous antigens comprise antigens associated with inflammatory diseases. Such antigens include, but are not limited to, those associated with Alzheimer's, arthritis, asthma, atherosclerosis, Crohn's disease, colitis, cystic fibrosis, dermatitis, diverticulitis, hepatitis, irritable bowel syndrome (IBS), lupus erythematous, muscular dystrophy, nephritis, Parkinson's, shingles and ulcerative colitis. Inflammatory disease associated antigens also include, for example, those associated with cardiovascular disease, chronic obstructive pulmonary disease (COPD), bronchiectasis, chronic cholecystitis, tuberculosis, Hashimoto's thyroiditis, sarcoidosis, silicosis.

In some cases, the endogenous antigens can be those associated with non-autoimmune inflammatory bowel disease, post-surgical adhesions, coronary artery disease, hepatic fibrosis, acute respiratory distress syndrome, acute inflammatory pancreatitis, endoscopic retrograde cholangiopancreatography-induced pancreatitis, burns, atherogenesis of coronary, cerebral and peripheral arteries, appendicitis, cholecystitis, diverticulitis, visceral fibrotic disorders, wound healing, skin scarring disorders (keloids, hidradenitis suppurativa), granulomatous disorders (sarcoidosis, primary biliary cirrhosis), asthma, pyoderma gandrenosum, Sweet's syndrome, Behcet's disease, or primary sclerosing cholangitis.

### Immunomodulators

### Exogenous Immunomodulators

Exogenous immunomodulators include, but are not limited to, statins; mTOR inhibitors, such as rapamycin or a rapamycin analog; TGF-β signaling agents; TGF-β receptor agonists; histone deacetylase (HDAC) inhibitors; corticosteroids; inhibitors of mitochondrial function, such as rotenone; P38 inhibitors; NF-κβ inhibitors; lectin receptor (e.g. CD22) ligands; adenosine receptor agonists; prostaglandin E2 agonists; phosphodiesterase inhibitors, such as phosphodiesterase 4 inhibitor; proteasome inhibitors; kinase inhibitors; G-protein coupled receptor agonists; G-protein coupled receptor antagonists; glucocorticoids; retinoids; cytokine inhibitors; cytokine receptor inhibitors; cytokine receptor activators; peroxisome proliferator-activated receptor antagonists; peroxisome proliferator-activated receptor agonists; histone deacetylase inhibitors; calcineurin inhibitors; phosphatase inhibitors and oxidized ATPs. Immunomodulators also include IDO, vitamin D3, cyclosporine A, aryl hydrocarbon receptor inhibitors, resveratrol, azathiopurine, 6-mercaptopurine, aspirin, niflumic acid, estriol, tripolide, interleukins (e.g., IL-1, IL-10), cyclosporine A, siRNAs targeting cytokines or cytokine receptors.

Examples of statins include atorvastatin (LIPITOR®, TORVAST®), cerivastatin, fluvastatin (LESCOL®, LESCOL® XL), lovastatin (MEVACOR®, ALTOCOR®, ALTOPREV®), mevastatin (COMPACTIN®), pitavastatin (LIVALO®, PIAVA®), rosuvastatin (PRAVACHOL®, SELEKTINE®, LIPOSTAT®), rosuvastatin (CRESTOR®), and simvastatin (ZOCOR®, LIPEX®).

Examples of mTOR inhibitors include rapamycin and analogs thereof (e.g., CCL-779, RAD001, AP23573, C20-methallylrapamycin (C20-Marap), C16-(S)-butylsulfonamidorapamycin (C16-BSrap), C16-(S)-3-methylindolerapamycin (C16-iRap) (Bayle et al. Chemistry & Biology 2006, 13:99-107)), AZD8055, BEZ235 (NVP-BEZ235), chrysophanic acid (chrysophanol), deforolimus (MK-8669), everolimus (RAD0001), KU-0063794, PI-103, PP242, temsirolimus, and WYE-354 (available from Selleck, Houston, TX, USA).

Examples of TGF-β signaling agents include TGF-β ligands (e.g., activin A, GDF1, GDF11, bone morphogenic proteins, nodal, TGF-βs) and their receptors (e.g., ACVR1B, ACVR1C, ACVR2A, ACVR2B, BMPR2, BMPR1A, BMPR1B, TGFβRI, TGFβRII), R-SMADS/co-SMADS (e.g., SMAD1, SMAD2, SMAD3, SMAD4, SMAD5, SMAD8), and ligand inhibitors (e.g, follistatin, noggin, chordin, DAN, lefty, LTBP1, THBS1, Decorin).

Examples of inhibitors of mitochondrial function include atractyloside (dipotassium salt), bongkrekic acid (triammonium salt), carbonyl cyanide m-chlorophenylhydrazone, carboxyatractyloside (e.g., from *Atractylis gummifera*)*,* CGP-37157, (-)-Deguelin (e.g., from *Mundulea sericea*), F16, hexokinase II VDAC binding domain peptide, oligomycin, rotenone, Ru360, SFK1, and valinomycin (e.g., from *Streptomyces fulvissimus*) (EMD4Biosciences, USA).

Examples of P38 inhibitors include SB-203580 (4-(4-Fluorophenyl)-2-(4-methylsulfinylphenyl)-5-(4-pyridyl)1H-imidazole), SB-239063 (trans-1-(4hydroxycyclohexyl)-4-(fluorophenyl)-5-(2-methoxy-pyrimidin-4-yl) imidazole), SB-220025 (5-(2amino-4-pyrimidinyl)-4-(4-fluorophenyl)-1-(4-piperidinyl)imidazole)), and ARRY-797.

Examples of NF (e.g., NK-κβ) inhibitors include IFRD1, 2-(1,8-naphthyridin-2-yl)-Phenol, 5-aminosalicylic acid, BAY 11-7082, BAY 11-7085, CAPE (Caffeic Acid Phenethylester), diethylmaleate, IKK-2 Inhibitor IV, IMD 0354, lactacystin, MG-132 [Z-Leu-Leu-Leu-CHO], NFκB Activation Inhibitor III, NF-κB Activation Inhibitor II, JSH-23, parthenolide, Phenylarsine Oxide (PAO), PPM-18, pyrrolidinedithiocarbamic acid ammonium salt, QNZ, RO 106-9920, rocaglamide, rocaglamide AL, rocaglamide C, rocaglamide I, rocaglamide J, rocaglaol, (R)-MG-132, sodium salicylate, triptolide (PG490), wedelolactone.

Examples of lectin receptor ligands include CD22 ligands such as the 9-azido-9-deoxy-sialyloligosaccharides and other CD22 ligands disclosed in B. E. Collins et. al. "High-Affinity Ligand Probes of CD22 Overcome the Threshold Set by cis Ligands to Allow for Binding, Endocytosis, and Killing of B Cells" J. Immunol September 1, 2006 vol. 177 no. 5 2994-3003; and the ligands disclosed in G-J Boons, "Liposomes Modified by Carbohydrate Ligands can Target B cells for the Treatment of B-Cell Lymphomas" Expert Rev Vaccines. 2010 November; 9(11): 1251-1256).

Examples of adenosine receptor agonists include CGS-21680 and ATL-146e.

Examples of prostaglandin E2 agonists include E-Prostanoid 2 and E-Prostanoid 4.

Examples of phosphodiesterase inhibitors (non-selective and selective inhibitors) include caffeine, aminophylline, IBMX (3-isobutyl-1-methylxanthine), paraxanthine, pentoxifylline, theobromine, theophylline, methylated xanthines, vinpocetine, EHNA (erythro-9-(2-hydroxy-3-nonyl)adenine), anagrelide, enoximone (PERFAN™), milrinone, levosimendon, mesembrine, ibudilast, piclamilast, luteolin, drotaverine, roflumilast (DAXAS™, DALIRESP™), sildenafil (REVATION®, VIAGRA®), tadalafil (ADCIRCA®, CIALIS®), vardenafil (LEVITRA®, STAXYN®), udenafil, avanafil, icariin, 4-methylpiperazine, and pyrazolo pyrimidin-7-1.

Examples of proteasome inhibitors include bortezomib, disulfiram, epigallocatechin-3-gallate, and salinosporamide A.

Examples of kinase inhibitors include bevacizumab, BIBW 2992, cetuximab (ERBITUX®), imatinib (GLEEVEC®), trastuzumab (HERCEPTIN®), gefitinib (IRESSA®), ranibizumab (LUCENTIS®), pegaptanib, sorafenib, dasatinib, sunitinib, erlotinib, nilotinib, lapatinib, panitumumab, vandetanib, E7080, pazopanib, mubritinib.

Examples of glucocorticoids include hydrocortisone (cortisol), cortisone acetate, prednisone, prednisolone, methylprednisolone, dexamethasone, betamethasone, triamcinolone, beclometasone, fludrocortisone acetate, deoxycorticosterone acetate (DOCA), and aldosterone.

Examples of retinoids include retinol, retinal, tretinoin (retinoic acid, RETIN-A®), isotretinoin (ACCUTANE®, AMNESTEEM®, CLARAVIS®, SOTRET®), alitretinoin (PANRETIN®), etretinate (TEGISON™) and its metabolite acitretin (SORIATANE®), tazarotene (TAZORAC®, AVAGE®, ZORAC®), bexarotene (TARGRETIN®), and adapalene (DIFFERIN®).

Examples of cytokine inhibitors include IL1ra, IL1 receptor antagonist, IGFBP, TNF-BF, uromodulin, Alpha-2-Macroglobulin, Cyclosporin A, Pentamidine, and Pentoxifylline (PENTOPAK®, PENTOXIL®, TRENTAL®).

Examples of peroxisome proliferator-activated receptor antagonists include GW9662, PPARγ antagonist III, G335, T0070907 (EMD4Biosciences, USA).

Examples of peroxisome proliferator-activated receptor agonists include pioglitazone, ciglitazone, clofibrate, GW1929, GW7647, L-165,041, LY 171883, PPAPγ activator, Fmoc-Leu, troglitazone, and WY-14643 (EMD4Biosciences, USA).

Examples of histone deacetylase inhibitors include hydroxamic acids (or hydroxamates) such as trichostatin A, cyclic tetrapeptides (such as trapoxin B) and depsipeptides, benzamides, electrophilic ketones, aliphatic acid compounds such as phenylbutyrate and valproic acid, hydroxamic acids such as vorinostat (SAHA), belinostat (PXD101), LAQ824, and panobinostat (LBH589), benzamides such as entinostat (MS-275), CI994, and mocetinostat (MGCD0103), nicotinamide, derivatives of NAD, dihydrocoumarin, naphthopyranone, and 2-hydroxynaphaldehydes.

Examples of calcineurin inhibitors include cyclosporine, pimecrolimus, voclosporin, and tacrolimus.

Examples of phosphatase inhibitors include BN82002 hydrochloride, CP-91149, calyculin A, cantharidic acid, cantharidin, cypermethrin, ethyl-3,4-dephostatin, fostriecin sodium salt, MAZ51, methyl-3,4-dephostatin, NSC 95397, norcantharidin, okadaic acid ammonium salt from prorocentrum concavum, okadaic acid, okadaic acid potassium salt, okadaic acid sodium salt, phenylarsine oxide, various phosphatase inhibitor cocktails, protein phosphatase 1C, protein phosphatase 2A inhibitor protein, protein phosphatase 2A1, protein phosphatase 2A2, sodium orthovanadate.

### Endogenous Immunomodulators

In certain cases, the immunomodulators are available endogenously. Endogenous immunomdulators are generated by a subject's own body, but are not repeatedly administered as part of the antigen-specific immunotherapeutic or as part of some other therapeutic intervention. Examples of endogenous immunomodulators comprise substances and/or combinations of substances involved in apoptosis or related signalling, substances and/or combinations of substances involved in T or B cell biology, and substances and/or combinations of substances involved in dendritic cell biology. In such cases, supply of the antigen through repeated administration of the antigen-specific immunotherapeutic can initiate, or sustain, a tolerogenic process specific to the antigen of interest.

Specific examples of endogenous immunomodulators include apoptotic erythrocytes (disclosed in S. Kontos et al., "Engineering antigens for in situ erythrocyte binding induces T-cell deletion" Proc Natl Acad Sci USA. 2013 Jan 2;110(1):E60-8); particular cytokine combinations generated when antigen is presented without also supplying molecules involved in immune cell stimulation (e.g. MHC I/II or costimulatory molecules) or without enabling immune cells (such as T cells, particularly naive T cells) to acquire effector function (disclosed in Published US Patent Application 2012/0076831 to Miller et al.); and cytokine combinations generated when antigen is presented in an MHC-antigen complex that induces profileration of tolerogenic antigen-specific T cells (disclosed in Published US Patent Application 2009/0155292 to Santamaria et. al.).

In some cases, a component of an antigen-specific immunotherapeutic, such as an antigen or immunomodulator, may be isolated. Isolated refers to the element being separated from its native environment and present in sufficient quantities to permit its identification or use. This means, for example, the element may be (i) selectively produced by expression cloning or (ii) purified as by chromatography or electrophoresis. Isolated elements may be, but need not be, substantially pure. Because an isolated element may be admixed with a pharmaceutically acceptable excipient in a pharmaceutical preparation, the element may comprise only a small percentage by weight of the preparation. The element is nonetheless isolated in that it has been separated from the substances with which it may be associated in living systems, i.e., isolated from other lipids or proteins. Any of the elements described herein may be isolated and included in the compositions and/or antigen-specific immunotherapeutic in isolated form.

### D. METHODS OF MAKING AND USING THE COMPOSITIONS AND RELATED METHODS

The antigen-specific immunotherapeutics can be prepared in a variety of ways, depending on the nature of the composition. Specific elements of such preparations may be known in the art. Preparation methods for certain preferred cases of the recited antigen-specific immunotherapeutics are presented below; other preparation methods for other cases may be found in the relevant literature.

Synthetic nanocarriers, useful in various embodiments of the present invention, may be prepared using a wide variety of methods known in the art. For example, synthetic nanocarriers can be formed by methods as nanoprecipitation, flow focusing using fluidic channels, spray drying, single and double emulsion solvent evaporation, solvent extraction, phase separation, milling, microemulsion procedures, microfabrication, nanofabrication, sacrificial layers, simple and complex coacervation, and other methods well known to those of ordinary skill in the art. Alternatively or additionally, aqueous and organic solvent syntheses for monodisperse semiconductor, conductive, magnetic, organic, and other nanomaterials have been described (Pellegrino et al., 2005, Small, 1:48; Murray et al., 2000, Ann. Rev. Mat. Sci., 30:545; and Trindade et al., 2001, Chem. Mat., 13:3843). Additional methods have been described in the literature (see, e.g., Doubrow, Ed., "Microcapsules and Nanoparticles in Medicine and Pharmacy," CRC Press, Boca Raton, 1992; Mathiowitz et al., 1987, J. Control. Release, 5:13; Mathiowitz et al., 1987, Reactive Polymers, 6:275; and Mathiowitz et al., 1988, J. Appl. Polymer Sci., 35:755; US Patents 5578325 and 6007845; P. Paolicelli et al., "Surface-modified PLGA-based Nanoparticles that can Efficiently Associate and Deliver Virus-like Particles" Nanomedicine. 5(6):843-853 (2010)).

Various materials may be encapsulated into synthetic nanocarriers as desirable using a variety of methods including but not limited to C. Astete et al., "Synthesis and characterization of PLGA nanoparticles" J. Biomater. Sci. Polymer Edn, Vol. 17, No. 3, pp. 247-289 (2006); K. Avgoustakis "Pegylated Poly(Lactide) and Poly(Lactide-Co-Glycolide) Nanoparticles: Preparation, Properties and Possible Applications in Drug Delivery" Current Drug Delivery 1:321-333 (2004); C. Reis et al., "Nanoencapsulation I. Methods for preparation of drug-loaded polymeric nanoparticles" Nanomedicine 2:8- 21 (2006); P. Paolicelli et al., "Surface-modified PLGA-based Nanoparticles that can Efficiently Associate and Deliver Virus-like Particles" Nanomedicine. 5(6):843-853 (2010). Other methods suitable for encapsulating materials into synthetic nanocarriers may be used, including without limitation methods disclosed in United States Patent 6,632,671 to Unger October 14, 2003.

In certain cases, synthetic nanocarriers are prepared by a nanoprecipitation process or spray drying. Conditions used in preparing synthetic nanocarriers may be altered to yield particles of a desired size or property (e.g., hydrophobicity, hydrophilicity, external morphology, "stickiness," shape, etc.). The method of preparing the synthetic nanocarriers and the conditions (e.g., solvent, temperature, concentration, air flow rate, etc.) used may depend on the materials to be coupled to the synthetic nanocarriers and/or the composition of the polymer matrix.

If particles prepared by any of the above methods have a size range outside of the desired range, particles can be sized, for example, using a sieve.

Elements (i.e., components) of the synthetic nanocarriers (such as moieties of which an immunofeature surface is comprised, targeting moieties, polymeric matrices, antigens, and immunomodulators) may be coupled to the overall synthetic nanocarrier, e.g., by one or more covalent bonds, or may be coupled by means of one or more linkers. Additional methods of functionalizing synthetic nanocarriers may be adapted from Published US Patent Application 2006/0002852 to Saltzman et al., Published US Patent Application 2009/0028910 to DeSimone et al., or Published International Patent Application WO/2008/127532 A1 to Murthy et al.

Alternatively or additionally, synthetic nanocarriers can be coupled to components, such as immunomodulators or antigens, directly or indirectly via non-covalent interactions. In non-covalent cases, the non-covalent coupling is mediated by non-covalent interactions including but not limited to charge interactions, affinity interactions, metal coordination, physical adsorption, host-guest interactions, hydrophobic interactions, TT stacking interactions, hydrogen bonding interactions, van der Waals interactions, magnetic interactions, electrostatic interactions, dipole-dipole interactions, and/or combinations thereof. Such couplings may be arranged to be on an external surface or an internal surface of a synthetic nanocarrier. In some cases, encapsulation and/or absorption is a form of coupling. In some cases, the described synthetic nanocarriers can be combined with an antigen by admixing in the same vehicle or delivery system. Pharmaceutical dosage forms of synthetic nanocarriers may be produced using traditional pharmaceutical methods.

Modified or expressed antigens may be prepared according to the references cited elsewhere herein. In particular, fusion constructs for modified antigens may be prepared using conventional protein production techniques, as disclosed in Kontos. Expressed antigens may be prepared using a variety of techniques, depending on how the nucleotide material that will serve as the template for protein expression is to be delivered. For instance, techniques for delivery of nucleotide material can be found depending on the delivery/dosage form of the material (e.g. naked DNA/RNA, liposomal delivery, gene gun, etc.).

Typical compositions and/or antigen-specific immunotherapeutics may comprise inorganic or organic buffers (e.g., sodium or potassium salts of phosphate, carbonate, acetate, or citrate) and pH adjustment agents (e.g., hydrochloric acid, sodium or potassium hydroxide, salts of citrate or acetate, amino acids and their salts) antioxidants (e.g., ascorbic acid, alpha-tocopherol), surfactants (e.g., polysorbate 20, polysorbate 80, polyoxyethylene9-10 nonyl phenol, sodium desoxycholate), solution and/or cryo/lyo stabilizers (e.g., sucrose, lactose, mannitol, trehalose), osmotic adjustment agents (e.g., salts or sugars), antibacterial agents (e.g., benzoic acid, phenol, gentamicin), antifoaming agents (e.g., polydimethylsilozone), preservatives (e.g., thimerosal, 2-phenoxyethanol, EDTA), polymeric stabilizers and viscosity-adjustment agents (e.g., polyvinylpyrrolidone, poloxamer 488, carboxymethylcellulose) and co-solvents (e.g., glycerol, polyethylene glycol, ethanol).

Compositions and/or antigen-specific immunotherapeutics can be formulated to comprise pharmaceutically acceptable excipients. The compositions and/or antigen-specific immunotherapeutics may be made using conventional pharmaceutical manufacturing and compounding techniques to arrive at useful dosage forms. Techniques suitable for use in practicing the present invention may be found in Handbook of Industrial Mixing: Science and Practice, Edited by Edward L. Paul, Victor A. Atiemo-Obeng, and Suzanne M. Kresta, 2004 John Wiley & Sons, Inc.; and Pharmaceutics: The Science of Dosage Form Design, 2nd Ed. Edited by M. E. Auten, 2001, Churchill Livingstone. In one case, synthetic nanocarriers are suspended in sterile saline solution for injection together with a preservative.

It is to be understood that the compositions and/or antigen-specific immunotherapeutics of the invention can be made in any suitable manner, and the invention is in no way limited to compositions and/or antigen-specific immunotherapeutics that can be produced using the methods described herein. Selection of an appropriate method may require attention to the properties of the particular moieties being associated.

In some cases, compositions and/or antigen-specific immunotherapeutics are manufactured under sterile conditions or are terminally sterilized. This can ensure that resulting compositions and/or antigen-specific immunotherapeutics are sterile and non-infectious, thus improving safety when compared to non-sterile compositions and/or antigen-specific immunotherapeutics. This provides a valuable safety measure, especially when subjects receiving compositions and/or antigen-specific immunotherapeutics have immune defects, are suffering from infection, and/or are susceptible to infection. In some cases, compositions and/or antigen-specific immunotherapeutics may be lyophilized and stored in suspension or as lyophilized powder depending on the formulation strategy for extended periods without losing activity.

### Administration

The compositions of the invention, including the antigen-specific immunotherapeutic as appropriate, can be administered by a variety of routes, including but not limited to subcutaneous, intranasal, oral, intravenous, intraperitoneal, intramuscular, transmucosal, transmucosal, sublingual, rectal, ophthalmic, pulmonary, intradermal, transdermal, transcutaneous or intradermal or by a combination of these routes. Routes of administration also include administration by inhalation or pulmonary aerosol. Techniques for preparing aerosol delivery systems are well known to those of skill in the art (see, for example, Sciarra and Cutie, "Aerosols," in Remington's Pharmaceutical Sciences, 18th edition, 1990, pp. 1694-1712).

The compositions and/or antigen-specific immunotherapeutics of the invention can be administered in effective amounts, such as the effective amounts described elsewhere herein. Doses of the compositions and/or antigen-specific immunotherapeutics can contain varying amounts of immunomodulators and/or antigens. The amount of immunomodulators and/or antigens present in the compositions and/or antigen-specific immunotherapeutics can be varied according to the nature of the antigens and/or immunomodulators, the therapeutic benefit to be accomplished, and other such parameters. In some cases, dose ranging studies can be conducted to establish optimal therapeutic amount of immunomodulators and/or antigens to be present in the compositions and/or antigen-specific immunotherapeutics. In some cases, the immunomodulators and/or antigens are present in the compositions and/or antigen-specific immunotherapeutics in an amount effective to generate a tolerogenic immune response to antigens of interest upon administration to a subject.

### Repeated Administration

The compositions and methods described herein can be used to induce or enhance a tolerogenic immune response and/or to suppress, modulate, direct or redirect an undesired immune response.

"Repeated administration" or "repeatedly administer"or "repeatedly administering" and the like means boosting or extending the persistence of a previously established immune tolerance or an effect that is characteristic of tolerance. Repeated administration can involve one administration or a short course of treatment at a time when the established tolerance is declining or at risk of declining. Repeated administration begins upon the next dose or doses of the antigen-specific therapeutic administered following administration of an initial dose of an antigen-specific immunotherapeutic. The initial antigen-specific immunotherapeutic administered may be the same or different (in terms of composition, dosing, etc.) from the antigen-specific immunotherapeutic administered during repeated administration. Repeated dosing is preferably performed 1 week to 10 years, and more preferably 1 to 12 months after an initial dose of the antigen-specific therapeutic or a previous repeated administration. Application of the invention may involve regular repeated administrations on a schedule of administrations that occur semiweekly, weekly, biweekly, or on any other regular schedule.

The compositions and/or antigen-specific immunotherapeutics may be administered at a variety of frequencies. In a preferred case, at least one administration of the compositions and/or antigen-specific immunotherapeutics within a repeated administration are sufficient to generate a pharmacologically relevant response. In more preferred cases, at least two administrations, at least three administrations, or at least four administrations, of the compositions and/or antigen-specific immunotherapeutics are utilized to ensure a pharmacologically relevant response within the overall repeated administration.

Prophylactic repeated administration of the compositions and/or antigen-specific immunotherapeutics can be initiated prior to the onset of disease, disorder or condition or therapeutic repeated administration can be initiated after a disorder, disorder or condition is established.

In some cases, administration of an immunomodulator is undertaken e.g., prior to administration of an exogenous antigen. In exemplary cases, immunomodulators are administered at one or more times including, but not limited to, 30, 25, 20, 15, 14, 13, 12, 11, 10, 9, 8, 7, 6, 5, 4, 3, 2, 1, or 0 days prior to administration of an exogenous antigen. In addition or alternatively, immunomodulators can be administered to a subject following exogenous antigen administration. In exemplary cases, immunomodulators are administered at one or more times including, but not limited to, 1, 2, 3, 4, 5, 6, 7, 8, 9, 10, 11, 12, 13, 14, 15, 20, 25, 30, etc. days following administration of an exogenous antigen.

### Demonstrating Protocols and Elements Thereof

Protocols, and elements making up the protocols, can be demonstrated in human or non-human subjects. In cases wherein protocols are demonstrated in non-human subjects, such protocols or elements thereof can be translated into human protocols. For example, test results from protocols carried out in rodents or non-human primates can suggest protocol elements such as frequency of repeated dosing, dose amounts of the antigen-specific immunotherapeutic, number of administrations of the the antigen-specific immunotherapeutic within each instance of repeated dosing, routes of administration, and variations of the above elements within each protocol. In one case, rodent and/or non-human primate protocol results can suggest a dose amount (including maximum and minimum doses that define a therapeutic window) that is then scaled for use in a human protocol, based on customary scaling techniques, such as alimetric scaling. Non-human protocol elements can also suggest optimal frequency of the repeated dosing that can be translated to human protocols; with certain cases having approximately the same frequency, and other cases having an adjusted frequency based on differences between the non-human species and humans.

Such non-human protocols, or protocol elements, can be selected based on results that showed non-induction of immunosuppression upon repeated administration. Such non-human protocols, or elements thereof, can be translated for use in humans, to provide an expected safety (and possibly efficacy) benefit in humans when the compositions and/or antigen-specific immunotherapeutics are repeatedly administered. Non-human protocols, or elements thereof, can be translated into human protocols, or elements thereof, using the techniques and considerations noted above, elsewhere herein, and generally in the art.

Another aspect of the disclosure relates to kits. In some cases, the kit comprises a dose or more than one dose of an antigen-specific immunotherapeutic as described herein. In such cases, the kit comprises more than one dose of an immunomodulator. The kit may also comprise or further comprise more than one dose of an antigen. The doses of immunomodulator and/or antigen may be contained within separate containers or within the same container in the kit. In some cases, the container is a vial or an ampoule. In some cases, the doses of immunomodulator and/or antigen are contained within a solution separate from the containers, such that the doses may be added to the container at a subsequent time. In some cases, the doses of immunomodulator and/or antigen are in lyophilized form each in a separate container or in the same container, such that they may be reconstituted at a subsequent time. In some cases, the kit further comprises instructions for reconstitution, mixing, administration, etc. In some cases, the instructions include a description of the methods described herein. Instructions can be in any suitable form, e.g., as a printed insert or a label. In some cases, the kit further comprises one or more syringes.

### EXAMPLES

The following examples contain Reference Examples, which illustrate a number of other and further developments that are disclosed in the foregoing Reference Technical Disclosure and Detailed Disclosure, but are not presented or claimed as the invention as such, which is defined exclusively by the appended claims.

### Example 1: Demonstration of Non-Immunosuppressive Protocol Using Antigen-Specific Immunotherapeutic that is Repeatedly Administered

### Synthetic Nanocarrier Materials

Rapamycin was purchased from TSZ CHEM (185 Wilson Street, Framingham, MA 01702; Product Catalog # R1017). PLGA of approximately 25,000 Da was purchased from Lakeshore Biochemicals (756 Tom Martin Dr Birmingham, AL 35211). Product code 5050 DLG 2.5A. PLA-PEG-OMe block co-polymer with a methyl ether terminated PEG block of approximately 5,000 Da and PLA block of 48,000 Da was purchased from Lakeshore Biochemicals (756 Tom Martin Drive, Birmingham, AL 35211). Product Code 100 DL mPEG 5000 5CE. OPII.323 was purchased from BACHEM (3132 Kashiwa Street, Torrance, CA 90505; Lot Number # B06481). EMPROVE® Polyvinyl Alcohol 4-88, USP (85-89% hydrolyzed, viscosity of 3.4-4.6 mPa.s) was purchased from EMD Chemicals Inc. (480 South Democrat Road Gibbstown, NJ 08027. Part Number 1.41354).

### Synthetic Nanocarrier Method

Solutions were prepared as follows: Solution 1: PLGA at 100 mg/mL in methylene chloride. The solution was prepared by dissolving PLGA in pure methylene chloride. Solution 2: PLA-PEG at 100 mg/mL in methylene chloride. The solution was prepared by dissolving PLA-PEG in pure methylene chloride. Solution 3: Rapamycin at 50 mg/mL in methylene chloride. The solution was prepared by dissolving rapamycin in pure methylene chloride. Solution 4: OPII.323 at 20 mg/mL in 0.13 M HCl. The solution was prepared by dissolving OPII.323 in 0.13 M HCl. Solution 5: Polyvinyl alcohol at 50 mg/mL in 100 mM pH 8 phosphate buffer. Solution 6: 70 mM phosphate buffer, pH 8. A primary (W1/O) emulsion was first created by mixing Solutions 1 through 4. Solution 1 (0.75 mL), Solution 2 (0.25 mL), Solution 3 (0.20 mL), and Solution 4 (0.2 mL) were combined in a small glass pressure tube and sonicated at 50% amplitude for 40 seconds using a Branson Digital Sonifier 250.

A secondary (W1/O/W2) emulsion was then formed by adding Solution 5 (3.0 mL) to the primary emulsion, vortexing to create a crude dispersion, and then sonicating at 30% amplitude for 60 seconds using the Branson Digital Sonifier 250. The secondary emulsion was added to an open 50 mL beaker containing Solution 6 (30 mL) and stirred at room temperature for 2 hours to allow the dichloromethane to evaporate and the nanocarriers to form in suspension. A portion of the suspended nanocarriers was then washed by transferring the nanocarrier suspension to a centrifuge tube, spinning at 75,600 rcf for 35 minutes, removing the supernatant, and re-suspending the pellet in phosphate buffered saline. This washing procedure was repeated and then the pellet was re-suspended in PBS IX to achieve a nanocarrier suspension having a nominal concentration of 10 mg/mL on a polymer basis. The suspension was stored frozen at -20° C until use.

Nanocarrier size was determined by dynamic light scattering. The amount rapamycin in the nanocarrier was determined by HPLC analysis. The amount of OPII.323 in the nanocarrier was determined by HPLC analysis. The total dry-nanocarrier mass per mL of suspension was determined by a gravimetric method.

| Effective Diameter (nm) | TLR Agonist, % w/w | Antigen, % w/w |
|---|---|---|
| 211.6 | Rapamycin, 8.63 | OPII.323 peptide, 1.3 |

### ELISA: Measurement of Anti-OVA IgG

The level of IgG antibodies were measured. Blocker Casein in PBS (Thermo Fisher, Catalog #37528) was used as diluent. 0.05% Tween-20 in PBS was used as wash buffer, prepared by adding 10 ml of Tween-20 ((Sigma, Catalog #P9416-100mL) to 2 liters of a 10x PBS stock (PBS: OmniPur® 10X PBS Liquid Concentrate, 4L, EMD Chemicals, Catalog #6505) and 18 Liters of deionized water.

OVA protein at a stock concentration of 5 mg/ml was used as a coating material. A 1:1000 dilution to 5 µg/ml was used as a working concentration. Each well of the assay plates was coated with 100 µl diluted OVA per well, plates were sealed with sealing film (VWR catalog #60941-120), and incubated overnight at 4°C. Costar9017 96-well Flat bottom plates were used as assay plates, Costar9017.

Low-binding polypropylene 96-well plate or tubes were used as set-up plates, in which samples were prepared before being transferred to the assay plate. The setup plates did not contain any antigen and, therefore, serum antibodies did not bind to the plate during the setup of the samples. Setup plates were used for sample preparation to minimize binding that might occur during preparation or pipetting of samples if an antigen-coated plate was used to prepare the samples. Before preparing samples in the setup plate, wells were covered with diluent to block any non-specific binding and the plate was sealed and incubated at 4°C overnight.

Assay plates were washed three times with wash buffer, and wash buffer was completely aspirated out of the wells after the last wash. After washing, 300 µl diluent were added to each well of assay plate(s) to block non-specific binding and plates were incubated at least 2 hours at room temperature. Serum samples were prepared in the setup plate at appropriate starting dilutions. Starting dilutions were sometimes also prepared in 1.5 ml tubes using diluent. Appropriate starting dilutions were determined based on previous data, where available. Where no previous data was available, the lowest starting dilution was 1:40. Once diluted, 200 µl of the starting dilution of the serum sample was transferred from to the appropriate well of the setup plate.

An exemplary setup plate layout is described as follows: Columns 2 and 11 contained anti-Ovabumin monoclonal IgG2b isotype (AbCam, ab17291) standard, diluted to 1 µg/mL (1:4000 dilution). Columns 3-10 contained serum samples (at appropriate dilutions). Columns 1 and 12 were not used for samples or standards to avoid any bias of measurements due to edge effect. Instead, columns 1 and 12 contained 200 µl diluent. Normal mouse serum diluted 1:40 was used as a negative control. Anti-mouse IgG2a diluted 1:500 from 0.5mg/mL stock (BD Bioscience) was used as an isotype control.

Once all samples were prepared in the setup plate, the plate was sealed and stored at 4°C until blocking of the assay plates was complete. Assay plates were washed three times with wash buffer, and wash buffer was completely aspirated after the last wash. After washing, 100 µL of diluent was added to all wells in rows B-H of the assay plates. A 12-channel pipet was used to transfer samples from the setup plate to the assay plate. Samples were mixed prior to transfer by pipetting 150 µl of diluted serum up and down 3 times. After mixing, 150µl of each sample was transferred from the setup plate and added to row A of the respective assay plate.

Once the starting dilutions of each sample were transferred from the setup plate to row A of the assay plate, serial dilutions were pipetted on the assay plate as follows: 50 µl of each serum sample was removed from row A using 12-channel pipet and mixed with the 100 µl of diluent previously added to each well of row B. This step was repeated down the entire plate. After pipetting the dilution of the final row, 50 µl of fluid was removed from the wells in the final row and discarded, resulting in a final volume of 100 µl in every well of the assay plate. Once sample dilutions were prepared in the assay plates, the plates were incubated at room temperature for at least 2 hours.

After the incubation, plates were washed three times with wash buffer. Detection antibody (Goat anti-mouse anti-IgG, HRP conjugated, AbCam ab98717) was diluted 1:1500 (0.33 µg/mL) in diluent and 100 µl of the diluted antibody was added to each well. Plates were incubated for 1 hour at room temperature and then washed three times with wash buffer, with each washing step including a soak time of at least 30 seconds.

After washing, detection substrate was added to the wells. Equal parts of substrate A and substrate B (BD Biosciences TMB Substrate Reagent Set, catalog #555214) were combined immediately before addition to the assay plates, and 100 µl of the mixed substrate solution were added to each well and incubated for 10 minutes in the dark. The reaction was stopped by adding 50 µl of stop solution (2N H2SO4) to each well after the 10 minute period. The optical density (OD) of the wells was assessed immediately after adding the stop solution on a plate reader at 450 nm with subtraction at 570 nm. Data analysis was performed using Molecular Device's software SoftMax Pro v5.4. In some cases, a four-parameter logistic curve-fit graph was prepared with the dilution on the x-axis (log scale) and the OD value on the y-axis (linear scale), and the half maximum value (EC50) for each sample was determined. The plate template at the top of the layout was adjusted to reflect the dilution of each sample (1 per column).

### ELISA: Measurement of Anti-KLH IgG

The level of IgG antibodies were measured. Blocker Casein in PBS (Thermo Fisher, Catalog #37528) was used as diluent. 0.05% Tween-20 in PBS was used as wash buffer, prepared by adding 10 ml of Tween-20 ((Sigma, Catalog #P9416-100mL) to 2 liters of a 10x PBS stock (PBS: OmniPur® 10X PBS Liquid Concentrate, 4L, EMD Chemicals, Catalog #6505) and 18 Liters of deionized water.

KLH protein (Sigma, Catalog #H7127) at a stock concentration of 10 mg/ml was used as a coating material. A 1:2000 dilution to 5 µg/ml was used as a working concentration. Each well of the assay plates was coated with 100 µl diluted KLH per well, plates were sealed with sealing film (VWR catalog #60941-120), and incubated overnight at 4°C. Costar 9017 96-well Flat bottom plates were used as assay plates (Costar 9017).

Low-binding polypropylene 96-well plate or tubes were used as set-up plates, in which samples were prepared before being transferred to the assay plate. The setup plates did not contain any antigen and, therefore, serum antibodies did not bind to the plate during the setup of the samples. Setup plates were used for sample preparation to minimize binding that might occur during preparation or pipetting of samples if an antigen-coated plate was used to prepare the samples. Before preparing samples in the setup plate, wells were covered with diluent to block any non-specific binding and the plate was sealed and incubated at 4°C overnight.

Assay plates were washed three times with wash buffer, and wash buffer was completely aspirated out of the wells after the last wash. After washing, 300 µl diluent were added to each well of assay plate(s) to block non-specific binding and plates were incubated at least 2 hours at room temperature. Serum samples were prepared in the setup plate at appropriate starting dilutions. Starting dilutions were sometimes also prepared in 1.5 ml tubes using diluent. Appropriate starting dilutions were determined based on previous data, where available. Where no previous data was available, the lowest starting dilution was 1:40. Once diluted, 200 µl of the starting dilution of the serum sample was transferred from to the appropriate well of the setup plate.

An exemplary setup plate layout is described as follows: Columns 2 and 3 contained anti-KLH mouse monoclonal IgG1 isotype (AbCam, ab34607) standard, diluted to 0.2 µg/mL (1:5000 dilution from 1 mg/mL stock). Columns 4-12 contained serum samples (at appropriate dilutions). Column 1 was not used for samples or standards so the effect of diluent alone on the coating material could be assessed. Instead, column 1 contained 200 µl diluent. Normal mouse serum diluted 1:40 was used as a negative control. Anti-mouse IgG2a diluted 1:500 from 0.5mg/mL stock (BD Bioscience) was used as an isotype control.

Once all samples were prepared in the setup plate, the plate was sealed and stored at 4°C until blocking of the assay plates was complete. Assay plates were washed three times with wash buffer, and wash buffer was completely aspirated after the last wash. After washing, 100 µL of diluent was added to all wells in rows B-H of the assay plates. A 12-channel pipet was used to transfer samples from the setup plate to the assay plate. Samples were mixed prior to transfer by pipetting 150 µl of diluted serum up and down 3 times. After mixing, 150µl of each sample was transferred from the setup plate and added to row A of the respective assay plate.

Once the starting dilutions of each sample were transferred from the setup plate to row A of the assay plate, serial dilutions were pipetted on the assay plate as follows: 50 µl of each serum sample was removed from row A using 12-channel pipet and mixed with the 100 µl of diluent previously added to each well of row B. This step was repeated down the entire plate. After pipetting the dilution of the final row, 50 µl of fluid was removed from the wells in the final row and discarded, resulting in a final volume of 100 µl in every well of the assay plate. Once sample dilutions were prepared in the assay plates, the plates were incubated at room temperature for at least 2 hours.

After the incubation, plates were washed three times with wash buffer. Detection antibody (Goat anti-mouse anti-IgG, HRP conjugated, AbCam ab98717) was diluted 1:1500 (0.33 µg/mL) in diluent and 100 µl of the diluted antibody was added to each well. Plates were incubated for 1 hour at room temperature and then washed three times with wash buffer, with each washing step including a soak time of at least 30 seconds.

After washing, detection substrate was added to the wells. Equal parts of substrate A and substrate B (BD Biosciences TMB Substrate Reagent Set, catalog #555214) were combined immediately before addition to the assay plates, and 100 µl of the mixed substrate solution were added to each well and incubated for 10 minutes in the dark. The reaction was stopped by adding 50 µl of stop solution (2N H2SO4) to each well after the 10 minute period. The optical density (OD) of the wells was assessed immediately after adding the stop solution on a plate reader at 450 nm with subtraction at 570 nm. Data analysis was performed using Molecular Device's software SoftMax Pro v5.4. A four-parameter logistic curve-fit graph was prepared with the dilution on the x-axis (log scale) and the OD value on the y-axis (linear scale), and the half maximum value (EC50) for each sample was determined. The plate template at the top of the layout was adjusted to reflect the dilution of each sample (1 per column).

### Antigen-specific Tolerogenic Activity of Antigen-Specific Immunotherapeutics Under Repeated Administration

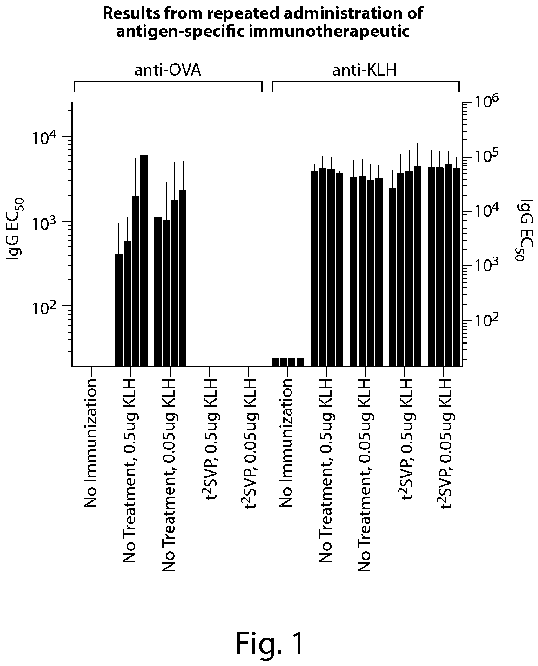

The purpose of this experiment was to assess the potential for immunosuppression of the effect of protocol of a repeatedly administered antigen-specific immunotherapeutic on nascent antibody responses by measuring antigen-specific immunogloblulins. One group of animals remained unimmunized as a control. All groups of animals were immunized using Chicken Ovalbumin (OVA) and CpG with 3 injections (initial treatment at d0, d14 and d28) in the right front and and hind footpads and with Key Limpet Hemocyanine (KLH) in the left front and hind foodpads. Antigen-specific immunotherapeutics (synthetic nanocarriers made according to the procedures above, and labeled "t²SVP") containing OPII were injected on day 0, and then repeatedly administered on days 14, 28, 42 and 56. For immunization, animals received 20 µl/limb of OVA+CpG, 12.5µg OVA+10 µg CpG (KLH as indicated in Fig. 1), both hind limbs s.c. t²SVP were diluted in such a manner that the same amounts of OVA₃₂₃₋₃₃₉ were injected in the treated groups. The results in Fig. 1 show that, following repeated administration of an antigen-specific immunotherapeutic, the titers against OVA are greatly affected by treatment with t²SVP (five left set of columns) but not the anti KLH titers (five right set of columns). For each group of animals treated differently the titers for days 21, 35, 49 and 63 (from left to right) are shown. Thus, the protocol was demonstrated not to induce immunosuppression upon repeated administration.

### Non-Immunosuppressive Antigen-specific Immunotherapeutic Repeated Administration Protocol (Prophetic)

In the practice of the present invention, this protocol, or elements thereof, would be used to generate a non-immunosuppressive protocol for use in other subjects. The dose amount element would be scaled, for instance, in humans by increasing the dose using alimetric scaling techniques to still preserve the non-immunosuppression of the underlying protocol established above.

### Example 2: PLP-Coupled Tolerogenic Synthetic Nanocarriers Utilizing Endogenous Antigen Repeated Administered (Prophetic)

PLP-coupled synthetic nanocarriers are prepared according to the methods laid out in Example 21 of Published US Patent Application 2012/0076831 to Miller et. al. ("Miller"). The synthetic nanocarriers are initially administered to SJL mice intravenously at a dose of 10 mg nanocarriers/kg body weight on day 0, and then repeatedly administered i.v. biweekly for 6 weeks following initial administration. Blood samples are taken at day 0, immediately prior to each repeated administration, and one week following the final repeat administration.

The blood samples are analyzed to establish KLH IgG titers using a KLH IgG ELISA procedure as generally set forth in Example 1 above. The absence of immunosuppression, as evidenced by KLH IgG tiers being above background in one or more of the samples taken following a repeated administration of the synthetic nanocarriers, may be noted.

Following an initial dose at 10 mg/kg, the synthetic nanocarriers are then repeatedly administered i.v. to human subjects in a dose amount scaled based on relative mass between mouse and human at the same dose of 10 mg/kg. Repeated dosing frequency is weekly for 3 weeks following the initial administration and monthly thereafter. The human subjects are monitored for clinical signs of opportunistic infections or other symptoms of a suppressed immune system.

### Reference Example 3: Nanogel-type Tolerogenic Synthetic Nanocarriers Utilizing Endogenous Antigen Repeatedly Administered (Prophetic)

Mycophenolic acid containing nanogel-type synthetic nanocarriers are prepared according to the methods disclosed in M. Look et. al. "Nanogel-based delivery of mycophenolic acid ameliorates systemic lupus erythematosus in mice" J Clin Invest. doi:10.1172/JCI65907 (2013). The synthetic nanocarriers are initially administered to C57BL/6 mice daily for 4 days at a dose of 0.625 mg of MPA per kilogram of animal body weight ("mpk") intravenously, and then repeatedly administered i.v. monthly for 6 months following initial administration. Blood samples are taken at day 0, immediately prior to each repeated administration, and one week following the final repeat administration.

The blood samples are analyzed to establish KLH IgG titers using a KLH IgG ELISA procedure as generally set forth in Example 1 above. The absence of immunosuppression, as evidenced by KLH IgG tiers being above background in one or more of the samples taken following a repeated administration of the synthetic nanocarriers, may be noted.

The synthetic nanocarriers are then repeatedly administered i.v. to human subjects in a dose amount, i.e. 0.625 mpk, scaled based on relative mass between mouse and human. The initial dose is daily for two days. Repeated dosing frequency is monthly for 6 months following the initial administration, at 0.625 mpk. The human subjects are monitored for clinical signs of opportunistic infections or other symptoms of a suppressed immune system.

### Reference Example 4: Antigen Fusion Protein Utilizing Endogenous Immunomodulator Repeatedly Administered (Prophetic)

A fusion protein that combined erythropoietin with a murine erythrocyte-specific single-chain Fv (scFv) antibody fragment is generated using the disclosure of Kontos et al., discussed elsewhere herein. The fusion protein is then initially administered i.v. to 12-wk-old female C57BL/6 mice daily for 3 days with the dose calculated to contain 10 µg of fusion protein per dose, and then repeatedly administered i.v. biweekly for 6 months following initial administration. Blood samples are taken at day 0, immediately prior to each repeated administration, and one week following the final repeat administration.

The blood samples are analyzed to establish KLH IgG titers using a KLH IgG ELISA procedure as generally set forth in Example 1 above. The absence of immunosuppression, as evidenced by KLH IgG tiers being above background in one or more of the samples taken following a repeated administration of the synthetic nanocarriers, may be noted.

A human fusion protein, including human erythropoietin and an scFV fragment specific for human erythrocytes is then generated. The fusion protein is then initially administered i.v. daily for three days to human subjects in a dose amount based on the mouse 10 µg dose, scaled based on the relative blood volume between mouse and human. The repeated dose is the same as the initial dose. Repeated dosing frequency is monthly for 6 months following the initial administration, at half the initial dose. The human subjects are monitored for clinical signs of opportunistic infections or other symptoms of a suppressed immune system.

### Reference Example 5: Tolerogenic Synthetic Nanocarriers Utilizing Exogenous mRNA Antigen and Exogenous Immunomodulator Repeatedly Administered (Prophetic)

A degradable synthetic nanocarrier system comprised of a pH-responsive poly((β-amino-ester) (PBAE) core and a phospholipid shell is prepared according to the disclosure of Su et al., "In vitro and in vivo mRNA delivery using lipid-enveloped pH responsive polymer nanoparticles" Mol Pharm. 2011 June 6; 8(3): 774-787 ("Su"). The double emulsion formulation strategy is pursued, and mycophenolic acid, present as a solution having an MPA concentration of 100 mg/ml is encapsulated in the primary emulsion and subsequently in the synthetic nanocarriers. See also Moon et al.," Interbilayer-Crosslinked Multilamellar Vesicles as Synthetic Vaccines for Potent Humoral and Cellular Immune Responses" Nat Mater. 2011 March ; 10(3): 243-251 for further encapsulation strategies that may be used. mRNA for EPO is then coupled to the synthetic nanocarriers, according to the methods generally disclosed by Su. Alternatively, mRNA encoding other therapeutic proteins, such as mRNA-based vaccines or protein replacements, as set forth in Su, may be utilized.

The synthetic nanocarriers are then initially administered via i.v. infusion to Rhesus monkeys with the dose calculated to contain 7 mg/kg of synthetic nanocarriers, and then repeatedly administered i.v. bimonthly for 6 months following initial administration. Blood samples are taken at day 0, immediately prior to each repeated administration, and one week following the final repeat administration.

The blood samples are analyzed to establish KLH IgG titers using a KLH IgG ELISA procedure as generally set forth in Example 1 above. The absence of immunosuppression, as evidenced by KLH IgG tiers being above background in one or more of the samples taken following a repeated administration of the synthetic nanocarriers, may be noted.

The synthetic nanocarriers are then repeatedly administered i.v. to human subjects in a dose amount based on doubling the initial 7 mg/kg dose (i.e. 14 mg/kg), scaled based on the relative mass between monkey and human. Repeated dosing frequency is monthly for 6 months following the initial administration. The human subjects are monitored for clinical signs of opportunistic infections or other symptoms of a suppressed immune system.

### Reference Example 6: Tolerogenic Synthetic Nanocarriers Utilizing Exogenous cDNA Antigen and Exogenous Immunomoulator Repeatedly Administered (Prophetic)

Genosphere-format synthetic nanocarriers are prepared. Rapamycin is encapsulated in the synthetic nanocarriers by dissolving rapamycin in ethanol and combining the rapamycin solution with the lipid solution to arrive at a calculated rapamycin load of 4% w/w, based on the weight of the dry ingredients added to the nanocarrier formulation. The DNA phase comprises a plasmid incorporating cDNA coding for erythropoietin ("EPO"), using a conventional plasmid technology that can be translated in both humans and non-human primates.

The synthetic nanocarriers are then initially administered via i.v. infusion daily for two days to Macaque monkeys with the dose calculated to contain 12 mg/kg of synthetic nanocarriers, and then repeatedly administered i.v. monthly for 6 months following initial administration starting at twice the initial dose (i.e 24 mg/kg) and then tapering by 25% every two months thereafter (18 mg/kg, 12 mg/kg, 6 mg/kg). Blood samples are taken at day 0, immediately prior to each repeated administration, and one week following the final repeat administration.

The blood samples are analyzed to establish KLH IgG titers using a KLH IgG ELISA procedure as generally set forth in Example 1 above. The absence of immunosuppression, as evidenced by KLH IgG tiers being above background in one or more of the samples taken following a repeated administration of the synthetic nanocarriers, may be noted.

The synthetic nanocarriers are then repeatedly administered i.v. to human subjects in a dose amount based on the tapering 24 mg/kg monkey dose (e.g. 24, 18, 12, and 6 mg/kg, with tapering occurring at two month intervals) which was repeatedly administered in monkeys, scaled based on the relative mass between monkey and human. Repeated dosing frequency is monthly for 6 months following the initial administration. The human subjects are monitored for clinical signs of opportunistic infections or other symptoms of a suppressed immune system.

### Reference Example 7: Tolerogenic Synthetic Nanocarriers Utilizing Exogenous mmRNA Antigen and Exogenous Immunomoulator Repeatedly Administered (Prophetic)

### Materials

Rapamycin is purchased from TSZ CHEM (185 Wilson Street, Framingham, MA 01702; Product Catalog # R1017). PLGA of approximately 25,000 Da is purchased from Lakeshore Biochemicals (756 Tom Martin Dr Birmingham, AL 35211). Product code 5050 DLG 2.5A. PLA-PEG-OMe block co-polymer with a methyl ether terminated PEG block of approximately 5,000 Da and PLA block of 48,000 Da is purchased from Lakeshore Biochemicals (756 Tom Martin Drive, Birmingham, AL 35211). Product Code 100 DL mPEG 5000 5CE. EMPROVE® Polyvinyl Alcohol 4-88, USP (85-89% hydrolyzed, viscosity of 3.4-4.6 mPa.s) is purchased from EMD Chemicals Inc. (480 South Democrat Road Gibbstown, NJ 08027. Part Number 1.41354).

Recombinant human Granulocyte-Colony Stimulating Factor (rhuG-CSF) modified mRNA is prepared according to the disclosure of Published US Patent Application 2013/0115272 to de Fougerolles et al.

### Method

Solutions are prepared as follows: Solution 1: PLGA at 100 mg/mL in methylene chloride. The solution is prepared by dissolving PLGA in pure methylene chloride. Solution 2: PLA-PEG at 100 mg/mL in methylene chloride. The solution is prepared by dissolving PLA-PEG in pure methylene chloride. Solution 3: Rapamycin at 50 mg/mL in methylene chloride. The solution is prepared by dissolving rapamycin in pure methylene chloride. Solution 4: rhuG-CSF modified mRNA at 20 mg/mL in 0.13 M HCl. The solution is prepared by dissolving the mmRNA in 0.13 M HCl. Solution 5: Polyvinyl alcohol at 50 mg/mL in 100 mM pH 8 phosphate buffer. Solution 6: 70 mM phosphate buffer, pH 8. A primary (W1/O) emulsion is first created by mixing Solutions 1 through 4.

Solution 1 (0.75 mL), Solution 2 (0.25 mL), Solution 3 (0.20 mL), and Solution 4 (0.2 mL) are combined in a small glass pressure tube and sonicated at 50% amplitude for 40 seconds using a Branson Digital Sonifier 250. The secondary (W1/O/W2) emulsion is then formed by adding Solution 5 (3.0 mL) to the primary emulsion, vortexing to create a crude dispersion, and then sonicating at 30% amplitude for 60 seconds using the Branson Digital Sonifier 250. The secondary emulsion is added to an open 50 mL beaker containing Solution 6 (30 mL) and stirred at room temperature for 2 hours to allow the dichloromethane to evaporate and the nanocarriers to form in suspension. A portion of the suspended nanocarriers is then washed by transferring the nanocarrier suspension to a centrifuge tube, spinning at 75,600 rcf for 35 minutes, removing the supernatant, and re-suspending the pellet in phosphate buffered saline. This washing procedure is repeated and then the pellet is re-suspended in PBS IX to achieve a nanocarrier suspension having a nominal concentration of 10 mg/mL on a polymer basis. The suspension is stored frozen at -20° C until use.

The synthetic nanocarriers are then initially administered via i.v. infusion daily for two days to Macaque monkeys with the dose calculated to contain 12 mg/kg of synthetic nanocarriers, and then repeatedly administered i.v. monthly for 6 months following initial administration at the same dose (i.e 12 mg/kg). Blood samples are taken at day 0, immediately prior to each repeated administration, and one week following the final repeat administration.

The blood samples are analyzed to establish KLH IgG titers using a KLH IgG ELISA procedure as generally set forth in Example 1 above. The absence of immunosuppression, as evidenced by KLH IgG tiers being above background in one or more of the samples taken following a repeated administration of the synthetic nanocarriers, may be noted.

The synthetic nanocarriers are then repeatedly administered i.v. to human subjects in a dose amount based on the 12 mg/kg dose which was repeatedly administered in monkeys, scaled based on the relative mass between monkey and human. Repeated dosing frequency is monthly for 6 months following the initial administration. The human subjects are monitored for clinical signs of opportunistic infections or other symptoms of a suppressed immune system.

### Reference Example 8: Tolerogenic Synthetic Nanocarriers Utilizing Exogenous mmRNA Antigen and Exogenous Immunomoulator Repeatedly Administered (Prophetic)

The procedures of Example 7 are repeated, except that the recombinant human Granulocyte-Colony Stimulating Factor (rhuG-CSF) modified mRNA is replaced with recombinant human erythropoietin (huEPO) modified mRNA. The human erythropoietin (rhuEPO) modified mRNA is prepared according to the disclosure of Published US Patent Application 2013/0115272 to de Fougerolles et al.

### Reference Example 9: Mesoporous Silica Nanoparticles with Coupled Ibuprofen (Prophetic)

Mesoporous SiO2 nanoparticle cores are created through a sol-gel process. Hexadecyltrimethyl-ammonium bromide (CTAB) (0.5 g) is dissolved in deionized water (500 mL), and then 2 M aqueous NaOH solution (3.5 mL) is added to the CTAB solution. The solution is stirred for 30 min, and then Tetraethoxysilane (TEOS) (2.5 mL) is added to the solution. The resulting gel is stirred for 3 h at a temperature of 80 °C. The white precipitate which forms is captured by filtration, followed by washing with deionized water and drying at room temperature. The remaining surfactant is then extracted from the particles by suspension in an ethanolic solution of HCl overnight. The particles are washed with ethanol, centrifuged, and redispersed under ultrasonication. This wash procedure is repeated two additional times.

The SiO2 nanoparticles are then functionalized with amino groups using (3-aminopropyl)-triethoxysilane (APTMS). To do this, the particles are suspended in ethanol (30 mL), and APTMS (50 µL) is added to the suspension. The suspension is allowed to stand at room temperature for 2 h and then is boiled for 4 h, keeping the volume constant by periodically adding ethanol. Remaining reactants are removed by five cycles of washing by centrifugation and redispersing in pure ethanol.

In a separate reaction, 1-4 nm diameter gold seeds are created. All water used in this reaction is first deionized and then distilled from glass. Water (45.5 mL) is added to a 100 mL round-bottom flask. While stirring, 0.2 M aqueous NaOH (1.5 mL) is added, followed by a 1% aqueous solution of tetrakis(hydroxymethyl)phosphonium chloride (THPC) (1.0 mL). Two minutes after the addition of THPC solution, a 10 mg/mL aqueous solution of chloroauric acid (2 mL), which has been aged at least 15 min, is added. The gold seeds are purified through dialysis against water.

To form the core-shell nanocarriers, the amino-functionalized SiO2 nanoparticles formed above are first mixed with the gold seeds for 2 h at room temperature. The gold-decorated SiO2 particles are collected through centrifugation and mixed with an aqueous solution of chloroauric acid and potassium bicarbonate to form the gold shell. The particles are then washed by centrifugation and redispersed in water. Ibuprofen is loaded by suspending the particles in a solution of sodium ibuprofen (1 mg/L) for 72 h. Free ibuprofen is then washed from the particles by centrifugation and redispersing in water.

### Reference Example 10: Liposomes Containing Cyclosporine A (Prophetic)

The liposomes are formed using thin film hydration. 1,2-Dipalmitoyl-sn-glycero-3-phosphocholine (DPPC) (32 µmol), cholesterol (32 µmol), and cyclosporin A (6.4 µmol) are dissolved in pure chloroform (3 mL). This lipid solution is added to a 50 mL round-bottom flask, and the solvent is evaporated on a rotary evaporator at a temperature of 60 °C. The flask is then flushed with nitrogen gas to remove remaining solvent. Phosphate buffered saline (2 mL) and five glass beads are added to the flask, and the lipid film is hydrated by shaking at 60 °C for 1 h to form a suspension. The suspension is transferred to a small pressure tube and sonicated at 60 °C for four cycles of 30s pulses with a 30 s delay between each pulse. The suspension is then left undisturbed at room temperature for 2 h to allow for complete hydration. The liposomes are washed by centrifugation followed by resuspension in fresh phosphate buffered saline.

### Example 11: Polymeric Nanocarrier Containing Polymer-Rapamycin Conjugate (Prophetic)

### Preparation of PLGA-rapamycin conjugate:

PLGA polymer with acid end group (7525 DLG1A, acid number 0.46 mmol/g, Lakeshore Biomaterials; 5 g, 2.3 mmol, 1.0 eq) is dissolved in 30 mL of dichloromethane (DCM). N,N-Dicyclohexylcarbodimide (1.2 eq, 2.8 mmol, 0.57 g) is added followed by rapamycin (1.0 eq, 2.3 mmol, 2.1 g) and 4-dimethylaminopyridine (DMAP) (2.0 eq, 4.6 mmol, 0.56 g). The mixture is stirred at rt for 2 days. The mixture is then filtered to remove insoluble dicyclohexylurea. The filtrate is concentrated to ca. 10 mL in volume and added to 100 mL of isopropyl alcohol (IPA) to precipitate out the PLGA-rapamycin conjugate. The IPA layer is removed and the polymer is then washed with 50 mL of IP A and 50 mL of methyl t-butyl ether (MTBE). The polymer is then dried under vacuum at 35 C for 2 days to give PLGA-rapamycin as a white solid (ca. 6.5 g).

### Preparation of nanocarrier containing PLGA-rapamycin conjugate and ovalbumin peptide (323-339):

Nanocarrier containing PLGA-rapamycin is prepared according to the procedure described in Example 1 as follows:
Solutions for nanocarrier formation are prepared as follows:
Solution 1: Ovalbumin peptide 323-339 @ 20 mg/mL in dilute hydrochloric acid aqueous solution. The solution is prepared by dissolving ovalbumin peptide in 0.13 M hydrochloric acid solution at room temperature. Solution 2: PLGA-rapamycin @ 100 mg/mL in methylene chloride. The solution is prepared by dissolving PLGA-rapamycin in pure methylene chloride. Solution 3: PLA-PEG @ 100 mg/mL in methylene chloride. The solution is prepared by dissolving PLA-PEG in pure methylene chloride. Solution 4: Polyvinyl alcohol @ 50 mg/mL in 100 mM pH 8 phosphate buffer.

A primary water-in-oil emulsion is prepared first. W1/O1 is prepared by combining solution 1 (0.2 mL), solution 2 (0.75 mL), and solution 3 (0.25 mL) in a small pressure tube and sonicating at 50% amplitude for 40 seconds using a Branson Digital Sonifier 250. A secondary emulsion (W1/O1/W2) is then prepared by combining solution 4 (3.0 mL) with the primary W1/O1 emulsion, vortexing for 10 s, and sonicating at 30% amplitude for 60 seconds using the Branson Digital Sonifier 250. The W1/O1/W2 emulsion is added to a beaker containing 70 mM pH 8 phosphate buffer solution (30 mL) and stirred at room temperature for 2 hours to allow the methylene chloride to evaporate and for the nanocarriers to form. A portion of the nanocarriers is washed by transferring the nanocarrier suspension to a centrifuge tube and centrifuging at 75,600×g and 4 °C for 35 min, removing the supernatant, and re-suspending the pellet in phosphate buffered saline. The washing procedure is repeated, and the pellet is re-suspended in phosphate buffered saline for a final nanocarrier dispersion of about 10 mg/mL.

### Reference Example 12: Preparation of Gold Nanocarriers (AuNCs) Containing Rapamycin (Prophetic)

### Preparation of HS-PEG-rapamycin:

A solution of PEG acid disulfide (1.0 eq), rapamycin (2.0-2.5 eq), DCC (2.5 eq) and DMAP (3.0 eq) in dry DMF is stirred at rt overnight. The insoluble dicyclohexylurea is removed by filtration and the filtrate is added to isopropyl alcohol (IPA) to precipitate out the PEG-disulfide-di-rapamycin ester and washed with IPA and dried. The polymer is then treated with tris(2-carboxyethyl)phosphine hydrochloride in DMF to reduce the PEG disulfide to thiol PEG rapamycin ester (HS-PEG-rapamycin). The resulting polymer is recovered by precipitation from IPA and dried as previously described and analyzed by H NMR and GPC.

### Formation of Gold NCs (AuNCs):

An aq. solution of 500 mL of 1 mM HAuC14 is heated to reflux for 10 min with vigorous stirring in a 1 L round-bottom flask equipped with a condenser. A solution of 50 mL of 40 mM of trisodium citrate is then rapidly added to the stirring solution. The resulting deep wine red solution is kept at reflux for 25-30 min and the heat is withdrawn and the solution is cooled to room temperature. The solution is then filtered through a 0.8 µm membrane filter to give the AuNCs solution. The AuNCs are characterized using visible spectroscopy and transmission electron microscopy. The AuNCs are ca. 20 nm diameter capped by citrate with peak absorption at 520 nm.

### AuNCs conjugate with HS-PEG-rapamycin:

A solution of 150 µl of HS-PEG-rapamycin (10 µM in 10 mM pH 9.0 carbonate buffer) is added to 1 mL of 20 nm diameter citrate-capped gold nanocarriers (1.16 nM) to produce a molar ratio of thiol to gold of 2500:1. The mixture is stirred at room temperature under argon for 1 hour to allow complete exchange of thiol with citrate on the gold nanocarriers. The AuNCs with PEG-rapamycin on the surface is then purified by centrifuge at 12,000g for 30 minutes. The supernatant is decanted and the pellet containing AuNC-S-PEG-rapamycin is then pellet washed with 1x PBS buffer. The purified Gold-PEG-rapamycin nanocarriers are then resuspend in suitable buffer for further analysis and bioassays.

### Reference Example 13: Liposomes Containing Rapamycin and Ovalbumin (Prophetic)

The liposomes are formed by thin film hydration. 1,2-Dipalmitoyl-sn-glycero-3-phosphocholine (DPPC) (32 µmol), cholesterol (32 µmol), and rapamycin (6.4 µmol) are dissolved in pure chloroform (3 mL). This lipid solution is added to a 10 mL glass tube and the solvent is removed under nitrogen gas stream and desiccated for 6 hr. under vacuum. Multilamellar vesicles are obtained by hydration of the film with 2.0 ml of 25 mM MOPS buffer pH 8.5, containing excess amount of Ovalbumin. The tube is vortexed until the lipid film is peeled of from the tube surface. To break the multilamellar vesicles into monolamellar, ten cycles of freezing (liquid nitrogen) and thawing (30°C water bath) are applied. The sample is then diluted to 1 ml in 25 mM MOPS buffer pH 8.5. Size of the resulting liposome is homogenized by extrusion by passing the sample 10 fold through a 200 nm pore polycarbonate filters. The resulting liposomes are then used for further analysis and bioassays.

### Reference Example 14: Various Additional Tolerogenic Synthetic Nanocarriers Utilizing Endogenous Antigen Repeatedly Administered (Prophetic)

The synthetic nanocarriers disclosed in Examples 9, 10 and 12 are initially administered to C57BL/6 mice intravenously daily for 4 days at a dose of synthetic nanocarriers that provides 30 µg of immunomodulator per dose, and then repeatedly administered i.v. monthly for 6 months following initial administration. Blood samples are taken at day 0, immediately prior to each repeated administration, and one week following the final repeat administration.

The blood samples are analyzed to establish KLH IgG titers using a KLH IgG ELISA procedure as generally set forth in Example 1 above. The absence of immunosuppression, as evidenced by KLH IgG tiers being above background in one or more of the samples taken following a repeated administration of the synthetic nanocarriers, may be noted.

The synthetic nanocarriers are then repeatedly administered i.v. to human subjects in a dose amount scaled based on relative blood volume between mouse and human. The initial dose is daily for three days. Repeated dosing frequency is monthly for 6 months following the initial administration, at the same dose as the initial dose. The human subjects are monitored for clinical signs of opportunistic infections or other symptoms of a suppressed immune system.

### Reference Example 15: Tolerogenic Synthetic Nanocarriers Utilizing Exogenous Antigen and Exogenous Immunomoulator Repeatedly Administered (Prophetic)

The synthetic nanocarriers of Examples 11 and 13 are initially administered via i.v. infusion daily for two days to female beagles with the dose calculated to contain 25 mg/kg of synthetic nanocarriers, and then repeatedly administered i.v. monthly for 6 months following initial administration at the same dose. Blood samples are taken at day 0, immediately prior to each repeated administration, and one week following the final repeat administration.

The blood samples are analyzed to establish KLH IgG titers using a KLH IgG ELISA procedure as generally set forth in Example 1 above. The absence of immunosuppression, as evidenced by KLH IgG tiers being above background in one or more of the samples taken following a repeated administration of the synthetic nanocarriers, may be noted.

The synthetic nanocarriers are then repeatedly administered i.v. to human subjects in a dose amount based on the 25 mg/kg repeatedly administed beagle dose, scaled based on the relative mass between beagles and humans. Repeated dosing frequency is monthly for 6 months following the initial administration. The human subjects are monitored for clinical signs of opportunistic infections or other symptoms of a suppressed immune system.

### Reference Example 16: Various Additional Tolerogenic Synthetic Nanocarriers Utilizing Exogenous Antigen Repeatedly Administered (Prophetic)

The synthetic nanocarriers disclosed in Examples 9, 10 and 12 are initially administered to C57BL/6 mice intravenously daily for 4 days at a dose of synthetic nanocarriers that provides 30 µg of immunomodulator per dose, and then repeatedly administered i.v. monthly for 6 months following initial administration. The recombinant human erythropoietin (rhuEPO) modified mRNA of Example 7 is administered concomitantly with the synthetic nanocarriers, specifically within 24 hours of each dose of synthetic nanocarriers. Sufficient mmRNA is administered to achieve 10 milliunits per milliliter of huEPO. Blood samples are taken at day 0, immediately prior to each repeated administration, and one week following the final repeat administration.

The blood samples are analyzed to establish KLH IgG titers using a KLH IgG ELISA procedure as generally set forth in Example 1 above. The absence of immunosuppression, as evidenced by KLH IgG tiers being above background in one or more of the samples taken following a repeated administration of the synthetic nanocarriers, may be noted.

The synthetic nanocarriers are then repeatedly administered i.v. to human subjects in a dose amount scaled based on relative blood volume between mouse and human. Modified mRNA coding for rhuEPO (as described above) is concomitantly dosed (in this case, within 24 hours) in an amount administered to achieve 10-20 milliunits per milliliter of rhuEPO in the human subjects. The initial dose is daily for three days. Repeated dosing frequency is monthly for 6 months following the initial administration, at the same dose as the initial dose. The human subjects are monitored for clinical signs of opportunistic infections or other symptoms of a suppressed immune system.

### Reference Example 17: Methotrexate (MTX) Leads to Antigen-specific Immunological Tolerance

C57BL/6 age-matched (5-6 weeks) female mice were injected 5 times intravenously in the tail vein with weekly injections of 25µg of an immunogenic, particulate form of chicken Ovalbumin (pOVA). One group of animals received 3 intraperitoneal injections of 200µg of MTX along with the 3 first antigen injections the same day and the 2 following days. The untreated group received only antigen whereas the treated group received 9 injections total of MTX i.p. (days 1-3, 7-9, 14-16). On the 4th and 5th injection days all animals received 20µg of keyhole limpet hemocyanine (KLH) subcutaneously in the hind limbs admixed to 2µg of CpG in addition to the i.v. injection of pOVA.

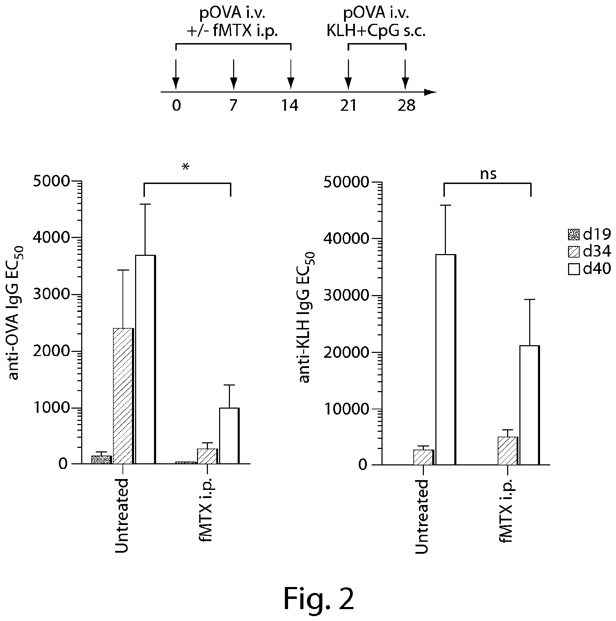

The anti-KLH and anti-OVA antibody responses were monitored in the blood in these animals at different time points. As shown in Fig. 2, in the absence of any treatment the animals developed a robust immune response against OVA. In contrast, administration of MTX blocked the antibody response and only minimal titers were detected, even after 5 injections with the antigen. When KLH and CpG were injected after the MTX treatments (from d21), a robust anti-KLH response could be detected in all groups. These results show that the immunosuppressive effect of MTX was lifted after day 21 and that the tolerogenic effect of MTX injections was restricted to the concomitantly administered antigen (OVA) (not the antigen provided after the MTX-treatment period).

Accordingly, these results show that repeated administrations of concomitant injections with MTX and antigen can prevent antigen-specific antibody formation without leading to immunosuppression. Thus, the protocol was demonstrated not to induce immunosuppression upon repeated administration.

### Reference Example 18: Methotrexate (MTX) Leads to Tolerance Induction to Multiple Antigens and Routes

In order to test whether multiple injections of MTX can lead to the establishment of immunological tolerance, C57BL/6 age-matched (5-6 weeks) female mice were injected intravenously in the tail vein with weekly injections of 200µg of keyhole limpet hemocyanin (KLH) and subcutaneously in the hind limbs with 25µg of a particulate form of chicken Ovalbumin (pOVA) admixed to 2µg of CpG oligodeoxynucleotides (ODN). With the first three antigen injections, a group of animals received 3 intraperitoneal injections of MTX on the same day and the following 2 days. All animals received 5 injections of antigen (d0, 7, 14, 21 and 28) and one group received 9 additional injections of MTX i.p. (days 1-3, 7-9, 14-16).

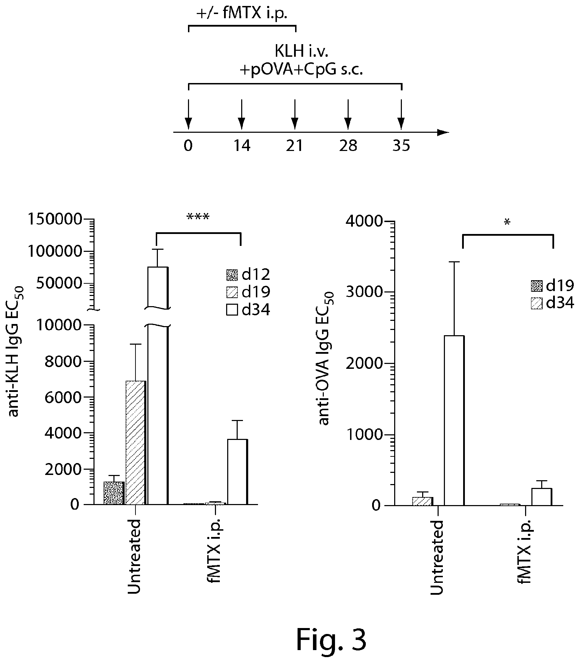

The anti-KLH and anti-OVA responses were monitored in the blood in these animals at different time points. As shown in Fig. 3, in the absence of any treatment the animals developed a robust immune response against KLH and OVA that can be measured by the anti-KLH and anti-OVA IgG antibody titers. In contrast, administration of MTX blocked both responses and the animals showed low titers even after 5 injections with the antigen.

Accordingly, these results show that repeated administrations of concomitant injections with MTX and antigen can prevent antigen-specific antibody formation without leading to immunosuppression. This was found with more than one antigen and with administration by different routes. Also, the protocol was demonstrated not to induce immunosuppression upon repeated administration.

### Example 19: OTI Model using ERY1-OVA

### Material:

Imject maleimide activated Ovalbumin: Thermoscientific, Product # 77126, Lot # OF185798, 10 mg, and ERY1 peptide (sequence: Trp-Met-Val-Leu-Pro-Trp-Leu-Pro-Gly-Thr-Leu-Asp-Gly-Gly-Ser-Gly-Cys-Arg-Gly-NH2) (SEQ ID NO: 1), CSBio, Product #CS11662, Lot# M613, MW 2001, TFA salt, 6 mg, ultrapure water and 1x PBS buffer were obtained.

### Methods

Imject maleimide activated OVA (10 mg) was dissolved in 2 mL of ultrapure water. To this solution was added a solution of ERY1 peptide (6 mg) in 0.6 mL of ultrapure water. The resulting solution was stirred at ambient temperature for 1 h and then at 8°C overnight. The slightly cloudy solution was diluted with 3 mL of 1xPBS and filtered through a 0.45 micron filter. The filtrate was then washed on a 10 KD MWCO Amicon-15 diafiltration tube with ultrapure water to remove excess ERY1 peptide. The concentrate was then diluted with ultrapure water to 1 mg/mL concentration (ca. 9 mL). The solution was finally filtered through a 0.2 micron filter to give the ERY1-OVA conjugate solution (1 mg/mL, ca. 9 mL). HPLC analysis confirmed the material as ERY1-OVA conjugate.

### Induction of CD8⁺ T Cell Tolerance using Erythrocyte Binding Peptide:

A syntheticl2-aa peptide (ERY1) discovered by phage display to bind to mouse glycophorin-A specifically ({Kontos, 2013 #8387}) is present uniquely on erythrocytes and can be conjugated to antigens to target erythrocytes and induce immunological tolerance. Without being bound by any particular theory, it is thought that binding to erythrocytes results in the handling of the antigen as an autoantigen during erythrocyte recycling and turnover.

To observe deletion or anergy of CD8⁺ T cells after administration of ERY1 conjugated to Ovalbumin (OVA), T cells were isolated from OTI+ transgenic animals that express a TCR recognizing a peptide of OVA in the MHC-CLI complex. These cells were transferred on day 1 into animals with a minor mismatch in the CD45 molecules (CD45.1+) to be able to track the donor cells (CD45.2+). One group of animals (n=3) remained untreated and unimmunized (naive), another group of animals remained untreated but was immunized (No Treatment) whereas the last group received OVA conjugated to ERY1 at days 0 and 5. All animals (except the first, "Naive" group) were immunized with OVA (10µg) and CpG ODN (10µg) in the hind limb subcutaneously on day 14. Five days later (day 19) all animals were sacrificed and the lymph nodes draining the site of immunization (popliteal) were excised and analyzed for the presence of the transferred CD45.2+ OTI+ cells by FACS.

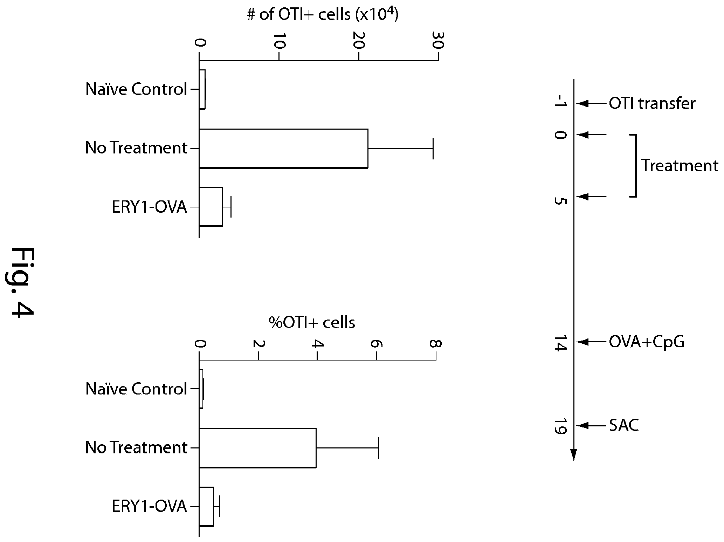

As shown in **Fig. 4****,** animals that remained naive have about 0.7x10⁴ OTI+ cells in their lymph nodes. Injections of OVA+CpG induce the activation and about a 30-fold expansion of these cells (21x10⁴) whereas treatment with ERY1-OVA complexes resulted in the complete inhibition of proliferative capacities of OTI cells. These results show that compositions described herein can annihilate the capacity of CD8⁺ T cells to recognize and get activated in the presence of the antigen. This illustrates the ability for a non-durable and antigen-specific downregulation of an immune response as a result of an antigen-specific immunotherapeutic as described herein.

## Claims

1. An antigen-specific immunotherapeutic composition for use in inducing antigen-specific tolerance, wherein repeated administration of the composition to a subject does not induce immunosuppression, and wherein the composition comprises polymeric synthetic nanocarriers coupled to an mTOR inhibitor and to an exogenous antigen, wherein the antigen is a therapeutic protein, or an antigenic fragment thereof, that is not coupled to the mTOR inhibitor, and wherein the mTOR inhibitor is rapamycin or a rapamycin analog.

2. The composition for use according to claim 1, wherein the load of the mTOR inhibitor on average is at least 95%, 97%, 98% or 99% (weight/weight).

3. The composition for use according to any one of the preceding claims, wherein the therapeutic protein is an enzyme.

4. The composition for use according to any one of the preceding claims, wherein the composition comprises more than one exogenous antigen.

5. The composition for use according to any one of the preceding claims, wherein the repeated administration occurs 1 week after an initial dose or a previous repeated administration of the composition.

6. The composition for use according to any one of the preceding claims, wherein the repeated administration occurs 2 weeks after an initial dose or a previous repeated administration of the composition.

7. The composition for use according to any one of the preceding claims, wherein the repeated administration occurs 1 to 12 months after an initial dose or a previous repeated administration of the composition.

8. The composition for use according to any one of the preceding claims, wherein the composition is comprised in a kit.

## Patentansprüche

1. Antigenspezifische immuntherapeutische Zusammensetzung zur Verwendung beim Induzieren einer antigenspezifischen Toleranz, wobei die wiederholte Verabreichung der Zusammensetzung an ein Subjekt keine Immunsuppression herbeiführt, und wobei die Zusammensetzung polymere synthetische Nanoträger umfasst, die an einen mTOR-lnhibitor und an ein exogenes Antigen gekoppelt sind, wobei das Antigen ein therapeutisches Protein oder ein antigenes Fragment davon ist, das nicht an den mTOR-lnhibitor gekoppelt ist und wobei der mTOR-lnhibitor Rapamycin oder ein Rapamycin-Analogon ist.

2. Zusammensetzung zur Verwendung nach Anspruch 1, wobei die Beladung des mTOR-Inhibitors im Durchschnitt wenigstens 95 %, 97 %, 98 % oder 99 % (Gewicht/Gewicht) beträgt.

3. Zusammensetzung zur Verwendung nach einem der vorhergehenden Ansprüche, wobei das therapeutische Protein ein Enzym ist.

4. Zusammensetzung zur Verwendung nach einem der vorhergehenden Ansprüche, wobei die Zusammensetzung mehr als ein exogenes Antigen umfasst.

5. Zusammensetzung zur Verwendung nach einem der vorhergehenden Ansprüche, wobei die wiederholte Verabreichung 1 Woche nach einer Anfangsdosis oder einer vorherigen wiederholten Verabreichung der Zusammensetzung erfolgt.

6. Zusammensetzung zur Verwendung nach einem der vorhergehenden Ansprüche, wobei die wiederholte Verabreichung 2 Wochen nach einer Anfangsdosis oder einer vorherigen wiederholten Verabreichung der Zusammensetzung erfolgt.

7. Zusammensetzung zur Verwendung nach einem der vorhergehenden Ansprüche, wobei die wiederholte Verabreichung 1 bis 12 Monate nach einer Anfangsdosis oder einer vorherigen wiederholten Verabreichung der Zusammensetzung erfolgt.

8. Zusammensetzung zur Verwendung nach einem der vorhergehenden Ansprüche, wobei die Zusammensetzung in einem Kit enthalten ist.

## Revendications

1. Composition immunothérapeutique spécifique à un antigène à utiliser pour induire une tolérance spécifique à un antigène, dans laquelle l'administration répétée de la composition à un sujet n'induit pas d'immunosuppression, et dans laquelle la composition comprend des nanoporteurs synthétiques polymères couplés à un inhibiteur de mTOR et à un antigène exogène, l'antigène étant une protéine thérapeutique, ou un fragment antigénique de celle-ci, qui n'est pas couplé à l'inhibiteur de mTOR, et l'inhibiteur de mTOR étant la rapamycine ou un analogue de la rapamycine.

2. Composition à utiliser selon la revendication 1, dans laquelle la charge de l'inhibiteur de mTOR est en moyenne d'au moins 95 %, 97 %, 98 % ou 99 % (poids / poids).

3. Composition à utiliser selon l'une quelconque des revendications précédentes, dans laquelle la protéine thérapeutique est une enzyme.

4. Composition à utiliser selon l'une quelconque des revendications précédentes, dans laquelle la composition comprend plus d'un antigène exogène.

5. Composition à utiliser selon l'une quelconque des revendications précédentes, dans laquelle l'administration répétée a lieu 1 semaine après une dose initiale ou une administration répétée précédente de la composition.

6. Composition à utiliser selon l'une quelconque des revendications précédentes, dans laquelle l'administration répétée a lieu 2 semaines après une dose initiale ou une administration répétée précédente de la composition.

7. Composition à utiliser selon l'une quelconque des revendications précédentes, dans laquelle l'administration répétée a lieu 1 à 12 mois après une dose initiale ou une administration répétée précédente de la composition.

8. Composition à utiliser selon l'une quelconque des revendications précédentes, dans laquelle la composition est comprise dans un kit.
